# FASCICULE DE BREVET EUROPEEN

(11) **EP 1 521 591 B1**
(45) Date de publication et mention de la délivrance du brevet: **24.03.2010**
(21) Numéro de dépôt: 03748201.5
(22) Date de dépôt: 04.07.2003
(51) Int. Cl.: A61K 38/29, A61K 38/17, A61K 39/395, A61P 35/00

(54) **UTILISATION D'UN ANTICORPS ANTI-PTHrP(34-53) COMME ANTAGONISTE DE LA PTHrP POUR TRAITER LE CARCINOME A CELLULES RENALES**
VERWENDUNG EINES ANTIKÖRPERS ANTI-PTHrP(34-53) ALS ANTAGONIST VON PTHrP ZUR BEHANDLUNG VON NIERENZELLKARZINOMA
USE OF ANTI-PTHrP(34-53) AS A PTHrP ANTAGONIST FOR TREATING RENAL CELL CARCINOMA

(30) Priorité: 05.07.2002 FR 0208501
(43) Date de publication de la demande: 13.04.2005
(73) Titulaire: Université de Strasbourg, 67081 Strasbourg (FR); INSTITUT NATIONAL DE LA SANTE ET DE LA RECHERCHE MEDICALE (INSERM), 75654 Paris Cédex 13 (FR); Hôpitaux Universitaires de Strasbourg, 67091 Strasbourg Cedex (FR)
(72) Inventeur: MASSFELDER, Thierry, F-67230 Witternheim (FR); LANG, Hervé, F-67000 Strasbourg (FR); SCHORDAN, Eric, F-67117 Handschuheim (FR); HELWIG, Jean-Jacques, F-67300 Schiltigheim (FR)
(74) Mandataire: Gallois, Valérie
(86) Numéro de dépôt international: PCT/FR2003/002084
(87) Numéro de publication internationale: WO 2004/004756

(56) Documents cités:
- EP-A- 1 197 225
- EP-A1- 1 283 057
- P.B. BURTON ET AL.: "Parathyroid hormone related peptide can function as an autocrine growth factor in human renal cell carcinoma." BIOCHEMICAL AND BIOPHYSICAL RESEARCH COMMUNICATIONS, vol. 167, no. 3, 30 mars 1990 (1990-03-30), pages 1134-1138, XP002237379 ORLANDO, FL, US
- W.M. SAVAGE ET AL.: "PTHrP assay in long term follow up of renal clear cell carcinoma, and demonstration of PTHrP mRNA in tumor tissue." JOURNAL OF ENDOCRINOLOGY, vol. 137, no. suppl., 1993, page P57 XP008015885
- S.R.J. HOARE ET AL.: "Specificity and stability of a new PTH1 receptor antagonist, mouse TIP(7-39)." PEPTIDES, vol. 23, no. 5, mai 2002 (2002-05), pages 989-998, XP002237380 cité dans la demande
- K. AKINO ET AL.: "PARATHYROID HORMONE-RELATED PEPTIDE IS A POTENT TUMOR ANGIOGENIC FACTOR" ENDOCRINOLOGY, BALTIMORE, MD, US, vol. 141, no. 11, novembre 2000 (2000-11), pages 4313-4316, XP001051188 ISSN: 0013-7227 cité dans la demande
- M. IWAMURA ET AL: "Parathyroid hormone-related protein is an independent prognostic factor for renal cell carcinoma" CANCER, vol. 86, no. 6, 15 September 1999 (1999-09-15), pages 1028-1034,

## Description

La présente invention a pour objet l'utilisation d'au moins un antagoniste de la PTHrP dans la préparation d'une composition pharmaceutique destinée à traiter le carcinome à cellules rénales (CCR) chez le mammifère et en particulier chez l'homme. De manière avantageuse, la présente invention présente un intérêt tout particulier pour inhiber ou diminuer la croissance tumorale et/ou la formation de métastases dans le cancer du rein et ses évolutions métastatiques, notamment au niveau du poumon et du foie.

La protéine apparentée à l'hormone parathyroïdienne (*parathyroid hormone-related protein*, ou PTHrP) est une poly-hormone. Elle a été découverte en 1987 à partir de tumeurs hypercalcémiantes chez l'homme et est reconnue aujourd'hui comme étant la principale responsable du syndrome d'hypercalcémie humorale maligne, un syndrome paranéoplasique associé à un grand nombre de tumeurs (Philbrick W. M. et al., Physiol. Rev, 76:127-73, 1996).

A côté des tumeurs, la PTHrP est exprimée de manière ubiquitaire par les tissus normaux, où elle apparaît notamment comme un nouveau facteur régulant localement la croissance, la différenciation, et la mort cellulaire. Son importance biologique est clairement mise en évidence par la mort précoce *in utero* des souris chez lesquelles le gène de la PTHrP ou celui de son récepteur (le récepteur commun PTH/PTHrP, ou RPTH1) ont été invalidés. Le rein n'échappe pas à cette règle : la PTHrP est exprimée dans toutes les composantes fonctionnels du rein (vaisseaux, glomérules, tubules) et y exerce des effets sur la prolifération et la différenciation cellulaire dans les conditions normales et pathologiques. Les inventeurs de la présente invention ont considérablement participé à la mise en évidence de ces propriétés.

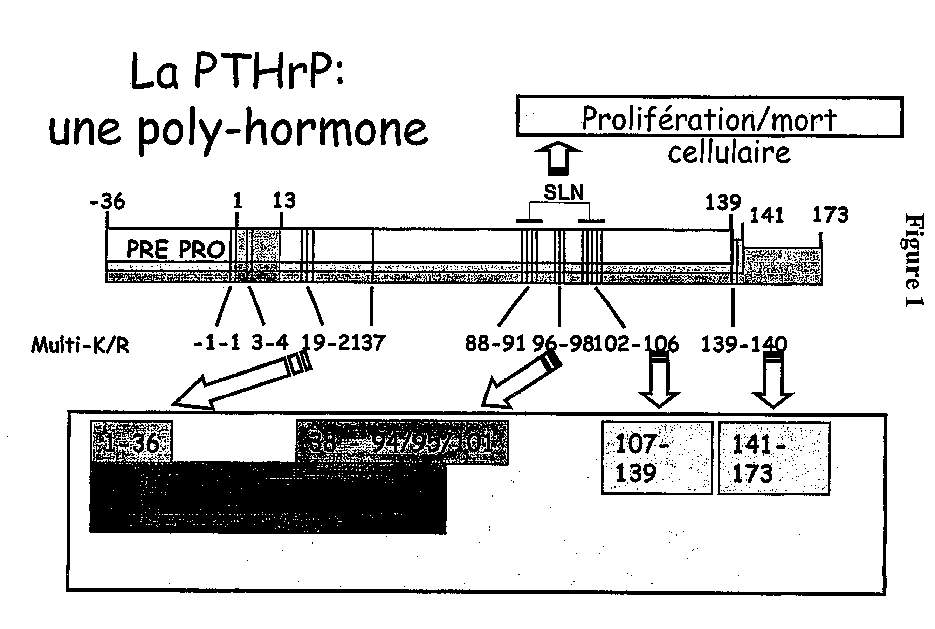

Il a été identifié trois isoformes de la PTHrP. Chez l'homme, le gène de la PTHrP (chromosome 12) code pour 3 isoformes de 139, 141 et/ou 173 acides aminés (seulement les formes de 139 et/ou de 141 acides aminés sont présentes chez les espèces inférieures : souris, rats, lapins, ...). Ces isoformes contiennent dans leur structure primaire un certain nombre de sites multi-basiques (multi-K/R) qui servent de sites post-traductionnelles de protéolyse permettant de générer plusieurs fragments tous biologiquement actifs: un fragment amino-terminal (1-36) qui contient une homologie de séquence limitée avec l'hormone parathyroïdienne (8 acides aminés sur les 13 premiers sont identiques entre hormone parathyroïdienne, nommée PTH, et PTHrP); une forme intermédiaire (38-94/95/101) et des formes carboxy-terminale (107-139) et probablement chez l'homme seulement une forme (141-173) (Philbrick et al., Physiol. Rev. 76: 127-173, 1996; Wu et al., J. Biol. Chem., 271: 24371-24381, 1996). En outre, des combinaisons des diverses formes énumérées ci-dessus peuvent également être des fragments de sécrétion. Ainsi, par exemple, au cours de l'hypercalcémie humorale maligne, un des fragments sécrétés par les tumeurs est un fragment amino-terminal contenant également la région intermédiaire dont on ne connaît toujours pas l'extrémité carboxy-terminale (fragment représenté par (1-?) sur la figure 1).

Les rôles biologiques de la PTHrP peuvent être résumés de la manière suivante :
- la myorelaxation des muscles lisses vasculaires et extra-vasculaires (fragments amino-terminaux),
- la régulation du transport trans-épithélial du calcium dans le rein, le placenta et la glande mammaire (fragments intermédiaires),
- rôle majeur au cours du développement qui a été mis en évidence par la létalité précoce *in utero* des souris invalidées pour le gène de la PTHrP ou de son récepteur (Philbrick et al., Physiol. Rev, 76 : 127-173, 1996),
- régulation de la prolifération et de la différenciation cellulaire et également de la mort cellulaire (tous les fragments).

Il est intéressant de mentionner que les séquences multi-basiques présentes dans la région intermédiaire de la molécule servent en réalité non seulement de sites post-traductionnels de protéolyse mais également de sites de localisation nucléaire (SLN) permettant à la molécule d'être dirigée vers le noyau cellulaire et de là de réguler la prolifération, la différenciation cellulaire, et la mort cellulaire par des mécanismes encore inconnus.

A l'heure actuelle, seul le récepteur liant les fragments amino-terminaux a été cloné, le récepteur commun PTH/PTHrP, dénommé RPTH1. Des données pharmacologiques existent quant à l'existence de récepteurs spécifiques des formes intermédiaires et carboxy-terminales.

En outre, il a récemment été montré que la PTHrP non seulement présente des activités angiogéniques sur les cellules endothéliales en culture, mais également qu'elle est capable de stimuler l'expression du facteur endothélial dérivé de l'endothélium (VEGF pour « vascular endothelial-derived growth factor ») dans diverses lignées cellulaires (Akino et al., Endocrinology, 141: 4313-4316, 2000 ; Esbrit et al., J. Am. Soc. Nephrol., 11:1085-1092, 2000).

Le carcinome à cellules rénales CCR est le groupe de tumeurs le plus fréquent du rein puisqu'il représente environ 80 à 85 % de l'ensemble des tumeurs du rein chez l'adulte. Le CCR peut se décomposer en 5 sous-types d'un point de vue pathologique, celui à cellules claires (CCC, incluant la variante à cellules acidophiles) étant la forme majoritaire puisqu'il représente environ 85% des CCR. Les autres sous-types par ordre décroissant d'incidence sont le carcinome papillaire (chromophiles), le carcinome à cellules chromophobes, le carcinome de Bellini et les carcinomes à cellules rénales non classées. Le carcinome rénal à cellules claires (CCC) qui représente presque 75 % des tumeurs malignes du rein et 2 % des décès liés au cancer en France et à travers le monde (7 000 cas/an et 3 500 décès en France en 2000 ; 200 000 cas/an et 100 000 décès/an à travers le monde) est une tumeur hyper-vascularisée qui trouve son origine au niveau de l'épithélium tubulaire proximal du rein. Les propriétés de la PTHrP décrites ci-dessus, avec d'une part la description d'un effet régulateur de la PTHrP sur la prolifération des cellules tubulaires proximales rénales (Garcia-Ocana et al, J. Bone Miner. Res., 10 :1875-1884, 1995 et Garcia-Ocana et al, J. Am. Soc. Nephrol., 10 : 238-244, 1999) et d'autre part les observations que la PTHrP immunoréactive est retrouvée dans les tumeurs chez 95 % des patients atteints de CCC (Iwamura et al, Cancer, 86 : 1028-1034, 1999), quelque soit leur calcémie, ont poussé les auteurs de la présente invention à rechercher le rôle du système PTHrP/RPTH1 dans le développement, la croissance et la néo-vascularisation de ces tumeurs. Ceci dans l'espoir de pouvoir mettre au point une thérapeutique efficace contre le CCR et en particulier contre le CCC, notamment contre sa forme métastatique, basée sur l'utilisation des composants du système PTHrP/RPTH1.

En effet, hormis la néphrectomie totale ou partielle applicable aux tumeurs localisées, aucune thérapie n'est actuellement efficace contre les formes métastatiques de ces tumeurs, celles-ci étant très résistantes aux traitements traditionnels qu'il s'agisse de l'hormonothérapie, de la chimiothérapie, de la radiothérapie et, à un degré moindre (taux de réponse inférieur à 15%), de l'immunothérapie (à base d'interleukines et d'interférons). Ainsi, actuellement aucune thérapie n'est réellement efficace contre le cancer du rein, alors que son incidence est en constante progression (45 % entre 1980 et 2000), et que le taux de survie est de 5 % à 5 ans en cas d'atteinte métastatique.

Un des aspects importants est que l'un des facteurs favorisants le carcinome à cellules rénales est la maladie de Von Hippel-Lindau (VHL) (Motzer et al., New Engl. J. Med., 335: 865-875, 1996). En effet, cette maladie est caractérisée par un fort taux, entre autres, de carcinomes à cellules rénales chez les patients. Ces carcinomes surviennent tôt, vers 30-40 ans. L'analyse génétique des patients a permis de découvrir un gène suppresseur de tumeur, le gène VHL (chromosome 3p), qui est inactif chez ces patients. Par la suite, les recherches ont permis de montrer que ce gène était également inactif chez la majorité des patients atteints d'un carcinome à cellules rénales non familial (dit sporadique), c'est-à-dire dans la population générale (Motzer et al., New Engl. J. Med., 335 : 865-875, 1996; Vogelzang et al., Lancet, 352 : 1691-1696, 1998).

Ainsi, les auteurs de la présente invention ont trouvé qu'in vitro sur diverses lignées cellulaires bien caractérisées issues de CCC humains, la PTHrP et le RPTH1 sont exprimés et que le blocage du système PTHrP/RPTH1 à l'aide d'anticorps dirigés contre la PTHrP ou d'un antagoniste compétitif des RPTH1 inhibe puissamment la prolifération cellulaire, mesurée en nombre de cellules et en incorporation de bromodéoxyuridine, ou BrdU. En effet, ces taux d'inhibition varient de 20 à 70 % suivant la lignée cellulaire et l'inhibiteur utilisé, un effet qui apparaît être la conséquence d'un double effet anti-prolifératif et de stimulation de la mort cellulaire tumorale.

De plus, in vivo, l'efficacité d'un traitement bloquant le système PTHrP/RPTH1 à l'aide d'un antagoniste compétitif des RPTH1 ou d'un anticorps anti-PTHrP a été testé chez la souris « nude » immunodéficiente. Il s'agit d'un modèle animal qui tolère l'implantation de tumeurs humaines permettant de tester l'efficacité de substances contre la croissance tumorale et la formation de métastases. Cette implantation peut se faire en sous-cutanée (implantation non invasive) ou de manière dite « orthotopique » (implantation invasive avec formation de métastases), c'est-à-dire en ce qui concerne le rein directement sous la capsule rénal. Après implantation en sous-cutanée des tumeurs humaines, les résultats montrent que le traitement sur une période de 25 jours par l'antagoniste des RPTH1 ou l'anticorps anti-PTHrP bloque la croissance des tumeurs implantées. Un traitement quotidien par l'anticorps anti-PTHrP a permis d'obtenir une regression complète de 70 % des tumeurs implantées et une regression partielle de 50 à 80 % des tumeurs restantes sur une période de 17 jours chez la souris nude.

Il est important de noter que les expériences montrent également que l'expression de la PTHrP est une cible directe ou indirecte du produit du gène suppresseur de tumeurs VHL (pour Von Hippel-Lindau), un gène défectueux dans les formes familiales de CCC (maladie de Von Hippel-Lindau) et dans la majorité (75 %) des CCC dits sporadiques (par opposition à familiaux). Dans ce schéma de régulation, l'absence de VHL actif dans les cellules tumorales est à l'origine d'une sur-expression de PTHrP. L'activité biologique fondamentale du VHL est la dégradation, en condition normoxique et via le protéasome, du facteur HIF-1 α (pour « Hypoxic-induced factor ») qui régule au niveau transcriptionnel l'expression des gènes impliqués dans la réponse cellulaire à l'hypoxie comme le VEGF, l'érythropoiétine et des facteurs de croissance et de différenciation cellulaire. Les conséquences d'une expression défectueuse du VHL sont donc une dédifférenciation cellulaire, une prolifération cellulaire accrue et la synthèse de facteurs angiogéniques à l'origine notamment de l'hypervascularisation des CCC. On comprend donc aisément l'importance du lien existant entre l'expression du VHL et celle de la PTHrP.

Ainsi ici est décrit plus particulièrement un système utilisant des antagonistes des RPTH1 pour bloquer la croissance des cellules tumorales de carcinome rénal à cellules claires humaines *in vitro* et *in vivo.*

Le cancer du rein se place en troisième position parmi les cancers de l'appareil urogénital après ceux de la prostate et de la vessie. Le CCC est plus fréquent chez l'homme que chez la femme avec un pic d'incidence entre 50 et 70 ans. Comme explicité ci-dessus, son incidence est en constante progression, et explique environ 2 % des décès liés au cancer en France et dans le monde. Le taux de mortalité est d'environ 30 %, mais le stade tumoral reste actuellement le meilleur facteur pronostic. Le taux de survie à 5 ans est de 70 % en cas de tumeurs purement intrarénales. Ce taux chute à 5 % en cas d'évolutions métastatiques (poumon, foie essentiellement), ce qui survient dans près de 30 % des cas en raison du caractère latent et relativement asymptomatique du CCC. Une des caractéristiques fondamentales de ces tumeurs est leur résistance à toutes les thérapies actuellement disponibles incluant l'hormonothérapie, la radiothérapie, la chimiothérapie et l'immunothérapie. Ainsi, dans le cas des cancers du rein métastatiques, la chimiothérapie et les traitements destinés à stimuler les défenses immunitaires (comme les interleukines et les interférons) donnent des résultats décevants avec des réponses ne dépassant pas 15 % et avec des effets secondaires très délétères sur les patients.

Ici décrit donc, une nouvelle thérapeutique basée sur l'utilisation d'antagonistes de PTHrP pour traiter les patients atteints de CCC, éventuellement avant et/ou après néphrectomie (préconisée dans les CCC, quelque soit le stade tumoral), notamment dans le but d'inhiber la croissance de la tumeur primitive et la croissance et/ou le développement des métastases associées, voire de faire régresser les tumeurs et les métastases.

Les études faites sur des antagonistes de la PTHrP sur l'animal - souris, rat et chien - dans un certain nombre de publications ne semblent pas montrer d'effets délétères sur les fonctions vitales, ce qui présente l'avantage de prévoir une certaine innocuité de ces composés.

Ainsi, la présente invention a pour objet l'utilisation d'un antagoniste de la PTHrP dans la préparation d'une composition pharmaceutique destinée à traiter, de manière préventive ou curative, un cancer du rein et en particulier le carcinome à cellules rénales (CCR) chez le mammifère et en particulier chez l'homme, ledit antagoniste étant un anticorps anti-PTHrP (34-53).

Elle concerne en particulier l'utilisation d'au moins un antagoniste de la PTHrP pour traiter le carcinome papillaire (chromophiles), le carcinome à cellules chromophobes, le carcinome de Bellini ou les carcinomes à cellules rénales non classées de CCR, de préférence ces composés permettent de traiter le carcinome à cellules claires (CCC).

De manière avantageuse, la présente invention présente un intérêt tout particulier pour inhiber ou diminuer la croissance tumorale et/ou la formation de métastases dans le cancer du rein et/ou ses évolutions métastatiques, notamment au niveau du poumon et du foie. Cette inhibition ou cette diminution peut aller, pour un fort pourcentage, jusqu'à la régression complète des tumeurs et des métastases associées au cancer du rein.

Il s'agit avantageusement d'une composition pharmaceutique pour le traitement (curatif) ou la prophylaxie (préventif) des cancers du rein, ledit composition étant un anticorps anti-PTHrP (34-53).

Les tumeurs malignes solides se répartissent en carcinomes (95%) et sarcomes (5%). Le développement des carcinomes et des sarcomes est corrélé à la mise en place d'une vascularisation des tumeurs, dénommée angiogénèse. Toute inhibition de cette angiogénèse tumorale permet de faire régresser, ou pour le moins de suspendre ou de retarder le développement des tumeurs. Or, il a été trouvé de manière surprenante que des anticorps anti-PTHrP et/ou des antagonistes des RPTH1 ont non seulement un effet sur les tumeurs, mais également un effet sur l'angiogénèse.

Les compositions pharmaceutiques ici décrit sont utilisables notamment pour le traitement de tumeurs malignes du cancer du rein, plus précisément le traitement de tumeurs malignes solides.

Elles sont également utilisables pour inhiber partiellement ou totalement l'angiogénèse tumorale.

L'invention concerne également une méthode de traitement du cancer du rein, en particulier des carcinomes à cellules claires, comprenant l'administration à un sujet, notamment humain, d'une dose efficace d'un antagoniste de la PTHrP ou d'une composition pharmaceutique le contenant, ledit composition étant un anticorps anti-PTHrP (34-53).

Le terme "un antagoniste de la PTHrP" inclut toute substance diminuant le ou les effets biologiques de la PTHrP, notamment ceux décrits ci-dessus. Une substance correspond à un composé ou à un mélange de composés.

Cette substance peut être en particulier une substance se liant au récepteur de la PTHrP inhibant de manière partielle, voire totale, la liaison de la PTHrP à son récepteur. De préférence, cette substance est un antagoniste du récepteur de la PTHrP, et de préférence un antagoniste compétitif de la PTHrP. Ces antagonistes incluent des peptides de la PTH ou de la PTHrP comprenant une substitution ou une délétion d'au moins un acide aminé de la séquence de la PTH et de la PTHrP ou une séquence partielle des peptides PTH ou PTHrP, éventuellement comprenant une substitution ou une délétion d'au moins un acide aminé de leur séquence. Des exemples spécifiques de substances antagonistes se liant aux récepteurs de PTHrP incluent la PTHrP (3-34), PTHrP (7-34), PTHrP (8-34), PTHrP (9-34), PTHrP (10-34), leurs amides ou leurs variants. Les variants présentent un remplacement, une délétion ou une addition d'au moins un acide aminé, tels que notamment (Asn10, Leu11, D-Trp12) PTHrP (7-34) amide (humain ou murin). En outre, parmi les polypeptides décrits ci-dessus, sont également compris ceux qui présentent une délétion, une substitution, une addition ou une insertion d'au moins un acide aminé de la séquence peptidique de la PTH ou de la PTHrP et qui présentent une activité antagoniste à la PTHrP. On peut également citer, comme antagoniste de la PTHrP, un dérivé du TIP (pour « tuberoinfundibular peptide »), tel que des peptides tronqués du TIP(1-39) (pour « tuberoinfundibular peptide » 1-39), notamment le TIP(7-39) et ses dérivés qui ont été décrits comme des antagonistes puissants des RPTH1 (Hoare et al, Peptides 23: 989-998, 2002).

L'antagoniste de la PTHrP peut être une substance non peptidique diminuant le ou les effets biologiques de la PTHrP.

L'antagoniste de la PTHrP peut être également une substance se liant à un ligand du récepteur de la PTHrP inhibant ainsi de manière partielle, voire totale, la liaison de PTHrP à son récepteur. Cette substance peut être choisie parmi les anticorps anti-PTHrP, et parmi ceux-ci de préférence un anticorps anti-PTHrP humanisé.

L'antagoniste de la PTHrP peut correspondre à une substance permettant d'augmenter la présence de VHL actif, diminuant ainsi le ou les effets biologiques de la PTHrP. Dans ce cadre, cette substance peut correspondre au produit du gène suppresseur de tumeurs VHL pouvant être obtenu en particulier par thérapie génique.

Par ailleurs, l'antagoniste de la PTHrP peut également correspondre à une substance permettant de réduire l'expression de la PTHrP. Cette substance se lie à l'ARNm ou au gène de PTHrP en inhibant, de manière partielle, voire totale, l'expression de PTHrP. Cette substance peut être par exemple un oligonucléotide antisense de la PTHrp, un ARNi, un facteur de transcription réprimant l'expression du gène de la PTHrP, ou une substance diminuant la stabilité de l'ARNm de la PTHrP.

La présente description inclut également la possibilité de mettre en oeuvre plusieurs sortes d'antagonistes de la PTHrP telles que définies ci-dessus pour traiter le cancer du rein. Ainsi, la présente description inclut, par exemple, une méthode de traitement incluant une thérapie génique telle que décrite ci-dessus et l'administration d'un antagoniste de la PTHrP, tel qu'un antagoniste du récepteur de la PTHrP ou un anticorps anti-PTHrP.

Le terme "un récepteur de la PTHrP " inclut un récepteur se liant au PTHrP, et inclut en particulier le récepteur PTH/PTHrP de type I (dénommé RPTH1).

Le terme "ligand" inclut une substance se liant à un enzyme ou un récepteur.

Le terme "une substance se liant à un ligand d'un récepteur de PTHrP inhibant la liaison entre le ligand et le récepteur" inclut une substance (e.g., un anticorps anti-PTHrP) qui inhibe la liaison d'un ligand (e.g., PTHrP) à un récepteur du PTHrP par liaison audit ligand du récepteur de PTHrP. Des exemples d'anticorps anti-PTHrP incluent des anticorps tels qu'un anticorps humanisé, un anticorps humain, un anticorps chimérique, un anticorps (tel que l'anticorps #23-57-137-1) obtenu à partir d'un hybridome (tel que l'hybridome #23-57-137-1) ou un fragment de ces anticorps et/ou une forme modifiée dudit fragment. L'anticorps peut être polyclonal, mais de préférence est monoclonal.

Le terme "un anticorps humanisé" inclut un anticorps comprenant une partie dérivée d'un anticorps humain et des régions déterminantes complémentaires (CDRs) dérivées d'un anticorps autre qu'humain (e.g. anticorps murin).

L'anticorps utilisé peut être produit par des méthodes connues de l'homme de l'art. De préférence, l'anticorps anti-PTHrP est un anticorps monoclonal dérivé d'un mammifère.

Cet anticorps inclut notamment ceux produits à partir d'un hybridome et ceux produits par des techniques génétiques à partir de cellules hôtes transformées avec un vecteur d'expression recombinant portant un gène codant pour l'anticorps. L'anticorps peut se lier à la PTHrP et empêcher la liaison de la PTHrP à son récepteur, bloquant ainsi le signal de transduction de PTHrP et par conséquent inhibant l'activité biologique de PTHrP.

A titre d'exemples, on peut notamment citer les anticorps anti-PTHrP(1-34) (humain, rat) de la société Bachem (Bachem Biochimie Sarl, Voisins-le-Bretonneux, France), l'anticorps anti-PTHrP(34-53) (Ab-2, humain) de la société Oncogene (France Biochem, Meudon, France), l'anticorps #23-57-137-1 (notamment décrit dans la demande de brevet EP1197225) et l'anticorps anti-PTHrP(107-139) (humain) obtenu par des méthodes conventionnelles de préparation d'anticorps.

L'hybridome produisant des anticorps monoclonaux peut être obtenu de la manière suivante. La PTHrP est utilisée en tant qu'antigène pour immunisation selon des méthodes conventionnelles d'immunisation. Les immunocytes qui en résultent sont fusionnés avec des cellules parentes connues selon des méthodes de fusions cellulaires conventionnelles, et les cellules produisant les anticorps sont alors criblées à partir des cellules fusionnées par des méthodes de criblage conventionnelles.

La PTHrP humaine utilisée en tant qu'agent antigène pour produire des anticorps est préparée par expression de la séquence du gène PTHrP décrit par Suva, L. J. et al., Science, 237, 893, 1987. Une séquence nucléotidique codant la PTHrP est insérée dans un vecteur d'expression connu, et une cellule hôte adaptée est transformée avec ce vecteur d'expression. La protéine PTHrP est ensuite isolée et purifiée à partir des cellules hôtes transformées ou à partir du surnageant obtenu à partir des cellules hôtes transformées, par des méthodes conventionnelles. La protéine PTHrP ainsi obtenue est utilisée comme antigène.

Selon un autre mode, un peptide correspondant à une séquence partielle du peptide PTHrP peut être chimiquement synthétisé et utilisé comme antigène. En particulier, une séquence partielle de 34-amino-acides de la région N-terminale de la séquence peptidique de PTHrP peut être chimiquement synthétisée et utilisée comme antigène.

Le mammifère à immuniser avec cet antigène peut être de tout type. Le mammifère est de préférence choisi en tenant compte de la compatibilité avec la cellule parente utilisée pour la fusion. On utilise généralement un rongeur (e.g., souris, rat ou hamster), un lapin ou un singe.

L'immunisation du mammifère avec l'antigène peut être réalisée par toute méthode connue en soi, par exemple, par injection intra péritonéale ou sous-cutanée de l'antigène au mammifère. Plus spécifiquement, l'antigène est dilué de manière appropriée dans une solution tampon-phosphate (PBS) ou une solution saline physiologique, la dilution en résultant ou la suspension est enuite mélangée en quantité appropriée avec un adjuvant conventionnel (e.g., Freund's complete adjuvant) pour obtenir une émulsion. L'émulsion est injectée au mammifère plusieurs fois à des intervalles de 4 à 21 jours. L'antigène peut éventuellement être attaché à un support approprié.

Après l'immunisation, le niveau antigénique du sérum est vérifié. Lorsque le niveau désiré est obtenu, les immunocytes sont isolés à partir du mammifère et fusionnées. L'immunocyte préféré utilisé est une cellule splénique.

La cellule parente utilisée pour la fusion est une cellule de myélome dérivé d'un mammifère. Cette cellule peut être toute lignée cellulaire connue et est par exemple P3 (P3x63Ag8.653) (J. Immunol. 123, 1548-1550, 1979), P3x63Ag8U.1 (Current Topics in Microbiology and Immunology 81, 1-7, 1978), NS-1 (Kohler, G. and Milstein, C. Eur. J. Immunol., 6, 511-519, 1976), MPC-11 (Margulies, D. H. et al., Cell, 8, 405-415, 1976), SP2/0 (Shulman, M. et al., Nature, 276, 269-270, 1978), FO (de St. Groth, S. F. et al., J. Immunol. Methods, 35, 1-21, 1980), S194 (Trowbridge, I. S., J. Exp. Med., 148, 313-323, 1978) or R210 (Galfre, G. et al., Nature, 277, 131-133, 1979).

La fusion est réalisée par toute méthode connue, telle que la méthode de Milstein et al. (Kohler, G. and Milstein, C., Methods Enzymol., 73, 3-46, 1981). En particulier, la fusion est réalisée dans un milieu de culture classique en présence d'un promoteur de fusion cellulaire. Le promoteur de fusion peut être le polyéthylène glycol (PEG) ou un virus connu à cet effet, tel que le virus de Sendai (hemagglutinating virus of Japan; HVJ). Si nécessaire, pour améliorer l'efficacité de la fusion, un additif peut être incorporé, tel que le diméthyl sulfoxide.

Le rapport des cellules immunocytes et du myélome peut varier dans une large mesure. A titre illustratif, les immunocytes peuvent être présents en quantité 1 à 10 fois plus importante que les cellules de myélome. Le milieu de culture utilisé pour la fusion est par exemple le milieu RPMI 1640 ou MEM approprié à la croissance des lignées cellulaires de myélome, ou tout autre milieu conventionnellement utilisé pour la culture de lignées cellulaires. Un sérum supplémentaire peut être ajouté au milieu, tel que le sérum de veau foetal (SVF, ou FCS pour « foetal calf serum »).

La fusion est donc réalisée en mélangeant les deux types cellulaires dans un milieu de culture, en ajoutant de préférence une solution de PEG au milieu généralement à une concentration de 30-60% (w/v). La solution est ensuite mélangée produisant ainsi les cellules fusionnées désirées, les hybridomes. Un milieu de culture approprié est ajouté ensuite à la solution obtenue puis l'ensemble est centrifugé pour retirer le surnageant. Les hybridomes obtenus sont sélectionnés par culture dans un milieu sélectif conventionnel, tel que le milieu hypoxanthine-aminopterin-thymidine (HAT), pour supprimer les cellules non désirées (non fusionnées). Une méthode de dilution est ensuite mise en oeuvre afin de cribler et de mono-cloner les hybridomes qui sécrètent l'anticorps désiré.

Selon une variante de production d'anticorps n'utilisant pas la technique des hybridomes, une immunisation d'un mammifère humain avec l'antigène tel que décrit ci-dessus peut être réalisée. En particulier, un lymphocyte humain peut être immunisé avec la PTHrP *in vitro*, puis fusionné avec une cellule de myélome humaine immortalisée, produisant ainsi un anticorps humain présentant une activité de liaison contre la PTHrP. Selon un autre mode, un anticorps humain contre PTHrP peut être préparé par injection de PTHrP en tant qu'antigène à un animal transgénique présentant le répertoire entier des gènes des anticorps humains, afin de produire des cellules produisant des anticorps anti-PTHrP, puis par immortalisation des cellules ainsi obtenues. Les cellules immortalisées ainsi préparées produisent l'anticorps humain attendu. Cette méthode est décrite en détail dans les brevets suivants (WO 96/33735 ; EP 822 830 ; US 6,150,584).

Des anticorps recombinants peuvent également être utilisés. Les anticorps recombinants peuvent être préparés par clonage d'un gène codant pour l'anticorps à partir de l'hybridome, en intégrant le gène dans un vecteur approprié, et en introduisant le vecteur dans une cellule hôte, pour produire l'anticorps à partir dé cette cellule hôte, selon des techniques de recombinaison génétique conventionnelles (Vandamme, A. M. et al., Eur. J. Biochem., 192, 767-775, 1990).

Un anticorps recombinant modifié artificiellement peut être également utilisé, notamment pour diminuer l'hétérogénéité contre le corps humain. Des anticorps chimériques ou humanisés peuvent ainsi être utilisés. Ces anticorps modifiés peuvent être produits par des méthodes connues.

L'anticorps utilisé peut être un fragment d'un anticorps ou une forme modifiée de ce fragment, ces substances doivent se lier à la PTHrP et inhiber l'activité biologique de la PTHrP. Ainsi, sont inclus les fragments d'anticorps, tels que Fab, Fab'2, CDRs, etc ou simple chaîne (ScFv).

Les fragments d'anticorps peuvent être produits en préparant des gènes pour ces fragments et en les exprimant dans des cellules hôtes appropriées.

Une forme modifiée d'un anticorps tel que décrit ci-dessus peut être également utilisée, par exemple, un anticorps anti-PTHrP conjugué à une molécule, telle que le polyéthylène glycol. Les anticorps modifiés peuvent être préparés par modification chimique des anticorps tels que décrits ci-dessus. Ces modifications chimiques sont connues de l'homme de l'art.

Pour la production des anticorps utilisés, tout système d'expression peut être utilisé, tel qu'un système cellulaire eucaryote ou procaryote. Les cellules eucaryotes incluent les lignées cellulaires établies (e.g., mammifères, insectes, champignons et levures). Les cellules procaryotes incluent les bactéries, tels que la bactérie E. Coli.

De préférence, l'anticorps utilisé est exprimé dans une cellule de mammifère, telle que les cellules CHO, COS, myélome, BHK, Vero ou HeLa.

La cellule hôte transformée est cultivée *in vitro* ou *in vivo* pour produire l'anticorps d'intérêt. Les cellules hôtes sont cultivées dans un milieu approprié conventionnel. Le milieu de culture peut choisi parmi le milieu DMEM, MEM, RPMI 1640 ou IMDM. Le milieu de culture peut comprendre en outre un sérum supplémentaire, tel que le sérum de veau foetal (FCS).

L'anticorps exprimé et produit tel que décrit ci-dessus peut être isolé à partir des cellules ou des animaux hôtes et purifié, par toute méthode connue en soi, notamment en utilisant une colonne d'affinité. Toute autre méthode connue pour isoler et purifier des anticorps peut être employée seule ou en combinaison, notamment à l'aide de chromatographes utilisant des colonnes, par des méthodes de filtration, d'ultrafiltration ou de dialyse.

La détermination de l'activité antigénique ou inhibitrice des anticorps ainsi préparés peut être réalisée en mettant en oeuvre toute méthode connu de l'homme de l'art, telle que notamment les méthodes ELISA (enzyme-linked immunosorbent assay), EIA (enzyme immunoassay), RIA (radioimmunoassay) ou les méthodes utilisant un anticorps fluorescent.

Les compositions pharmaceutiques comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions peuvent être administrés de différentes manières et sous différentes formes. Ainsi, ils peuvent être injectés par voie systémique ou orale, de préférence systémique, comme par exemple par voie intraveineuse, intra-musculaire, sous-cutanée, trans-dermique, intra-artérielle, etc. Pour les injections, les composés sont généralement conditionnés sous forme de suspensions liquides, qui peuvent être injectées au moyen de seringues ou de perfusions, par exemple. Il est entendu que le débit et/ou la dose injectée peuvent être adaptés par l'homme du métier en fonction du patient, de la pathologie, du mode d'administration, etc. Typiquement, les composés sont administrés à des doses pouvant varier entre 0,1 µg et 500 mg/kg de poids corporel, plus généralement de 0,01 à 10 mg/kg, typiquement entre 0,1 et 10 mg/kg. En outre, des injections répétées peuvent être réalisées, le cas échéant. D'autre part, les compositions peuvent comprendre, en outre, d'autres agents ou principes actifs.

### Légende des Figures

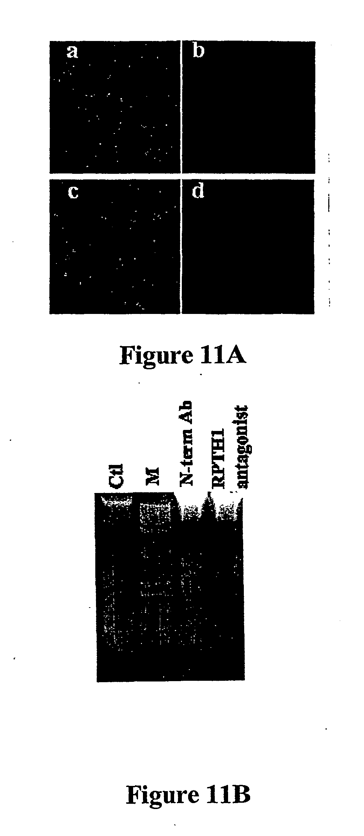

Figure 1 : schéma représentant le gène de la PTHrP et ses isoformes
Figure 2 : gels montrant l'expression de la PTHrP et du RPTH1 au niveau de l'ARNm dans 3 échantillons (numérotés 1, 2 et 3) des 3 lignées tumorales 786-0, UOK-126 et UOK-128. Expériences réalisées en RT-PCR.
Figure 3 : Fig. 3A: Dosage de la protéine PTHrP par une méthode radioimmunologique dans le milieu conditionné des 3 lignées tumorales 786-0, UOK-126 et UOK-128 en utilisant un anticorps reconnaissant spécifiquement la partie amino-terminale de la PTHrP. Fig. 3B : Mesure de l'expression du RPTH1 par Western blot dans les 3 lignées tumorales 786-0, UOK-126 et UOK-128 avec un anticorps dirigé contre RPTH1.
Figure 4 : La figure 4 représente l'effet des anticorps dirigés contre les diverses régions de la PTHrP sur la prolifération des cellules tumorales 786-0 *in vitro* mesurée sur le nombre de cellules (à gauche) et sur l'incorporation de BrdU (à droite).
Figure 5 : La figure 5 représente l'effet de l'antagoniste des RPTH1, la (Asn10, Leu11, D-Trp12)hPTHrP(7-34) amide (h pour humaine) à diverses concentrations sur la prolifération des cellules tumorales 786-0 *in vitro* mesurée sur le nombre de cellules (à gauche) et sur l'incorporation de BrdU (à droite).
Figure 6 : La figure 6 représente l'effet des anticorps dirigés contre les diverses régions de la PTHrP sur la prolifération des cellules tumorales UOK-126 *in vitro* mesurée sur le nombre de cellules (à gauche) et sur l'incorporation de BrdU (à droite).
Figure 7 : La figure 7 représente l'effet de l'antagoniste des RPTH1, la (Asn10, Leu11, D-Trp12)hPTHrP(7-34) amide à diverses concentrations sur la prolifération des cellules tumorales UOK-126 *in vitro* mesurée sur le nombre de cellules (à gauche) et sur l'incorporation de BrdU (à droite).
Figure 8 : La figure 8 représente l'effet des anticorps dirigés contre les diverses régions de la PTHrP sur la prolifération des cellules tumorales UOK-128 *in vitro* mesurée sur le nombre de cellules (à gauche) et sur l'incorporation de BrdU (à droite).
Figure 9 : La figure 9 représente l'effet de l'antagoniste des RPTH1, la (Asn10, Leu11, D-Trp12)h^{p}THrP(7-34) amide à diverses concentrations sur la prolifération des cellules tumorales UOK-128 *in vitro* mesurée sur le nombre de cellules (à gauche) et sur l'incorporation de BrdU (à droite).
Figure 10 : photographies des cellules 786-0 en culture dans un milieu RPMI 1640 sans sérum (avec 0,1% de BSA) et traitées soit en contrôle (à gauche) soit en présence de 10-6 M de l'antagoniste (Asn10, Leu11, D-Trp12)hPTHrP(7-34) amide (à droite) pendant 48h.
Figuré 11 : La figure 11 représente l'effet de l'antagoniste des RPTH1, la (Asn10, Leu11, D-Trp12)hPTHrP(7-34) amide sur l'apoptose des cellules tumorales 786-0. Fig. 11 A : micrographe de fluorescence des cellules contrôles (en haut) ou des cellules traitées par l'antagoniste de RPTH1 (en bas) avec l'acridine orange (AO : a, c) et le bromure d'éthidium (EB : b, d) (x 400). Fig. 11 B : test de fragmentation de l'ADN.

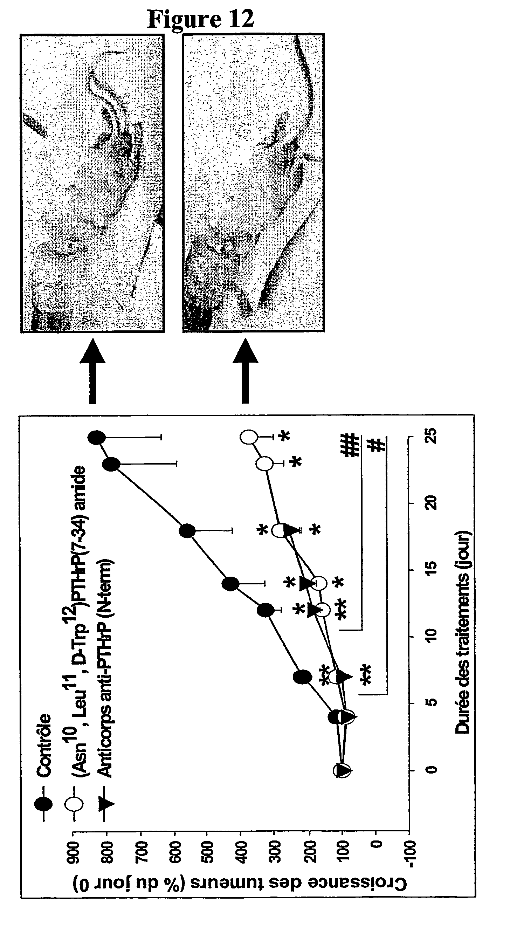

Figure 12 : La figure 12 représente la croissance des tumeurs des souris traitées soit en contrôle soit par l'antagoniste selon le protocole pilote décrit dans les exemples qui suivent. Les résultats de croissance sont exprimés en pourcentage par rapport au volume des tumeurs au jour 0 du début des traitements. Cette figure représente également les photographies : en haut une souris traitée en contrôle pendant 25 jours avec ses 4 tumeurs bien visibles et en bas une souris traitée pendant 25 jours par un antagoniste.

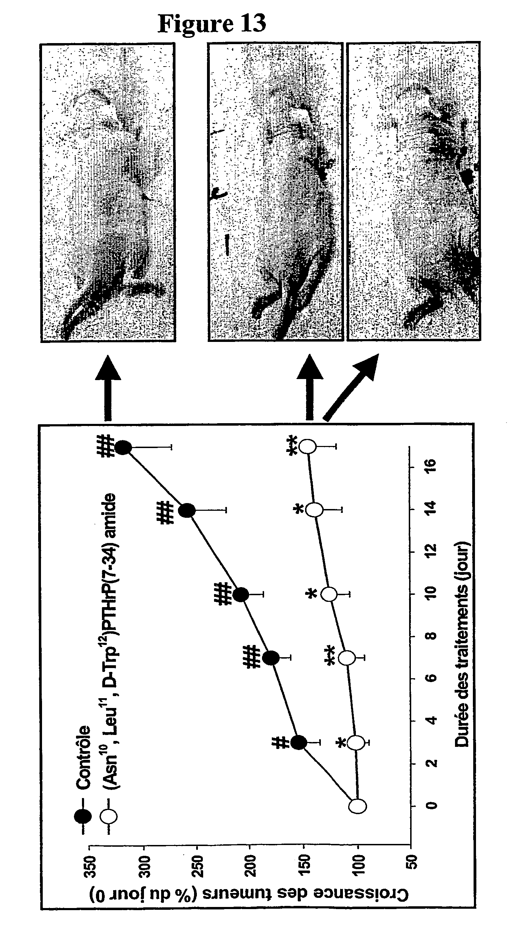

Figure 13 : La figure 13 représente la croissance des tumeurs des souris traitées soit en contrôle soit par l'antagoniste selon le protocole expérimental décrit ci-dessous. Les résultats de croissance sont exprimés en pourcentage par rapport au volume des tumeurs au jour 0 du début des traitements. Cette figure représente également les photographies : en haut une souris traitée en contrôle pendant 17 jours avec sa tumeur bien visible et en bas en exemple deux souris traitées pendant 17 jours par un antagoniste.

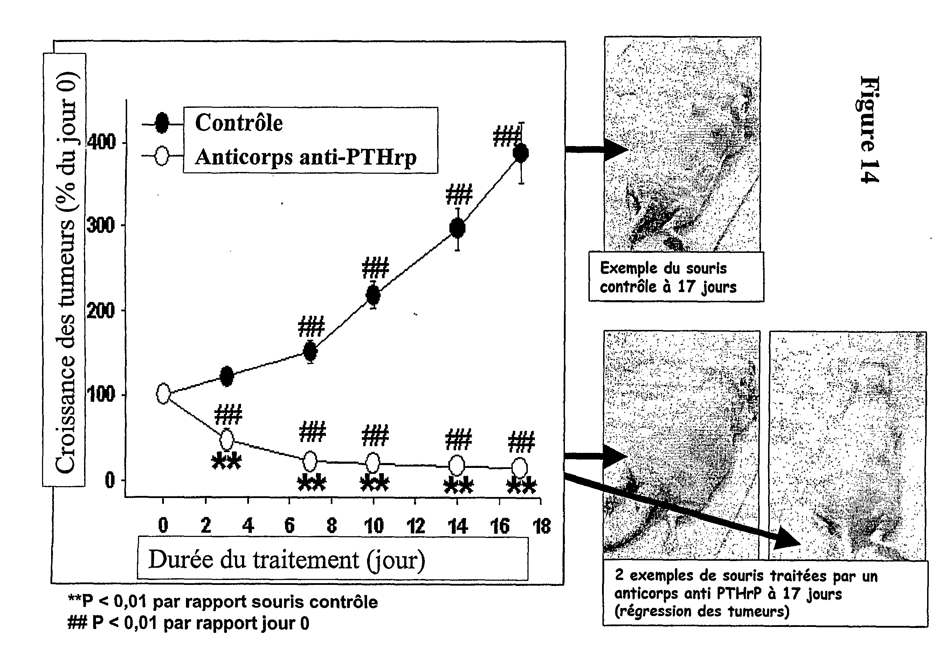

Figure 14 : La figure 14 représente la croissance des tumeurs des souris traitées soit par un anticorps dirigé contre l'extrémité N-terminale de PTHrP soit par des IgG non-spécifiques selon le protocole expérimental décrit ci-dessous. Les résultats de croissance sont exprimés en pourcentage par rapport au volume des tumeurs au jour 0 du début des traitements. Cette figure représente également les photographies : en haut une souris traitée en contrôle pendant 17 jours avec sa tumeur bien visible et en bas en exemple une souris traitée pendant 17 jours par un anticorps anti-PTHrP (N-term).

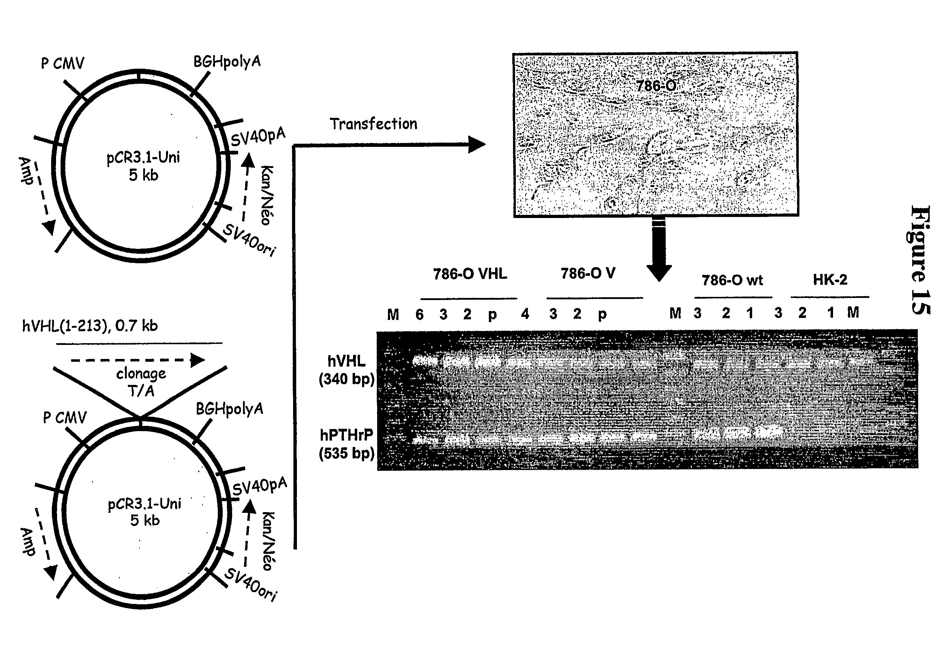

Figure 15 : Sur la figure 15 sont représentés à gauche les vecteurs pCR3.1-Uni seul ou codant pour le VHL(1-213). Ces vecteurs ont été transfectés dans les cellules 786-0 (cf. ci-dessous). Trois clones individuels ont été isolés après sélection au G418 ainsi que l'ensemble des clones pour les cellules transfectées par le vecteur seul (786-0 V, clones individuels 2, 3, 4 et clones mélangés p) ou par le vecteur codant pour le VHL (786-0 VHL, clones individuels 2, 3 et 6 et clones mélangés p). Parallèlement à ces clones, 3 échantillons de cellules 786-0 non transfectées (786-0 wt pour « wild-type ») et 3 échantillons de cellules HK-2 (cellules tubulaires proximales humaines normales) ont également été testées pour l'expression du VHL et de la PTHrP. Les gels de cette figure représentent l'expression du VHL obtenue par RT-PCR sur l'ARN total isolé des diverses cellules (bande à 340bp) selon le protocole décrit ci-dessous.

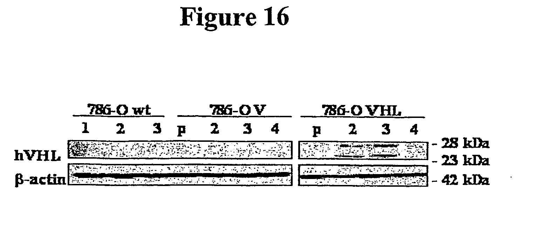

Figure 16 : Sur cette figure sont représentés les Western Blots des lysats de cellules 786-0 non transfectées et transfectées avec le vecteur seul ou le vecteur codant pour le VHL, ces lysats étant incubés en présence d'un anticorps dirigé contre l'épitope HA (pVHL) ou la β-actine.

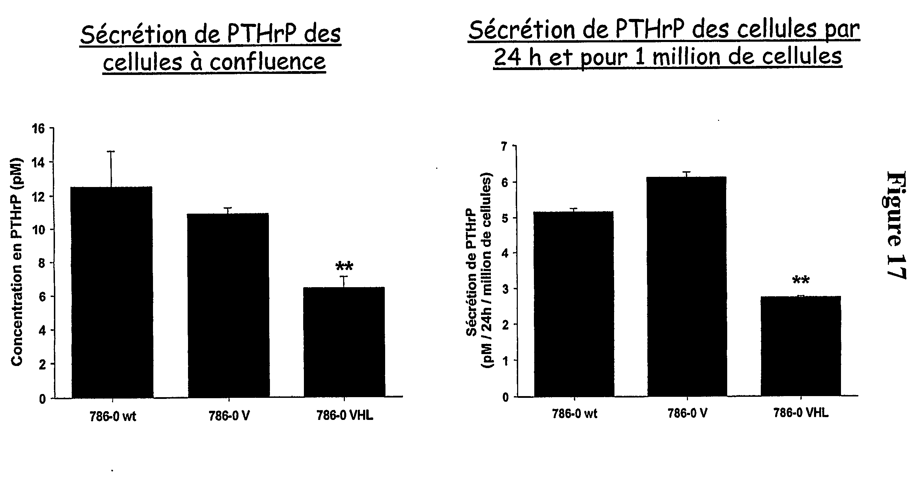

Figure 17 : La Figure 17 de gauche montre par RIA de la PTHrP que la production de PTHrP est diminuée de 50 % dans les cellules 786-0 transfectées avec le vecteur codant pour le VHL (786-0 VHL) par comparaison aux cellules non transfectées (786-0 wt) ou transfectées avec le vecteur seul (786-0 V). La figure 14 de droite représente la sécrétion de PTHrP en pM par 24h et par million de cellules.

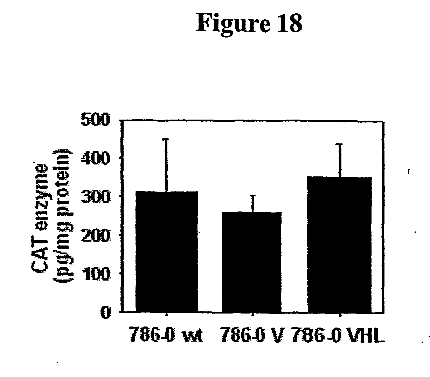

Figure 18 : Expression de CAT dans les cellules 786-0 non-transfectées, les clones 3 et 4 de cellules 786-0 transfectées par le vecteur, les clones 2 et 3 de cellules 786-0 transfectées par le vecteur codant pour le VHL, les clones étant transfectés transitoirement avec un plasmide d'expression codant pour la CAT sous la dépendance des promoteurs de hPTHrP (hPTHrP - CAT).

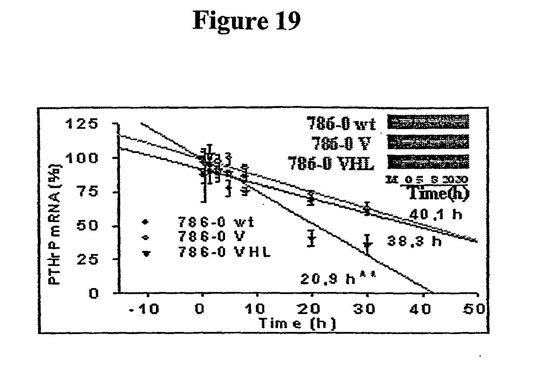

Figure 19 : Diminution du transcript ARNm de hPTHrP dans les cellules 786-0 non-transfectées, les clones 3 et 4 de cellules 786-0 transfectées par le vecteur, les clones 2 et 3 de cellules 786-0 transfectées par le vecteur codant pour le VHL.

D'autres aspects et avantages apparaîtront à la lecture des exemples qui suivent, qui doivent être considérés comme illustratifs et non limitatifs.

### EXEMPLES

Sauf mention contraire, les pourcentages donnés sont exprimés en poids. Rdt signifie rendement.

### Matériels.

L'antagoniste des RPTH1, la (Asn10, Leu11, D-Trp12)PTHrP(7-34) amide (humain, rat) et l'anticorps anti-PTHrP(1-34) (humain, rat) ont été obtenus de chez Bachem (Bachem Biochimie Sarl, Voisins-le-Bretonneux, France). L'anticorps anti-PTHrP(34-53) (Ab-2, humain) a été obtenu de chez Oncogene (France Biochem, Meudon, France) et l'anticorps anti-PTHrP(107-139) (humain) a été donné par le Dr P. Esbrit (Fundacion Jimenez Diaz, Madrid, Espagne).

### Lignées cellulaires et mise en culture.

La lignée cellulaire tumorale humaine 786-0 a été obtenue de ATCC (American Type Culture Collection, Manassas, VA, USA) dont le distributeur en Europe est la société LGC Promochem (Teddington, UK). Les lignées cellulaires tumorales humaines UOK-126 et UOK-128 ont été fournies par le Dr P. Anglard, (IGBMC, Strasbourg, France). La lignée cellulaire 786-0 (ATCC CRL-1932) correspond à des cellules issues d'un CCC humain métastatique (poumons) mutées sur le gène du VHL [perte d'1 allèle et délétion partielle du second produisant un VHL de 104 acides aminés, VHL (1-104), au lieu de VHL(1-213)] et tumorigéniques (Williams et al, In Vitro, 12: 623-627, 1976), la lignée cellulaire UOK-126 correspond à des cellules issues d'un CCC humain, perte d'hétérozygotie sur le chromosome 3p (Anglard et al, Cancer Res., 52: 348-356, 1992) et la lignée cellulaire UOK-128 correspond à des cellules issues d'un CCC humain, perte d'hétérozygotie sur le chromosome 3p (Anglard et al, Cancer Res., 52: 348-356, 1992).

Les cellules 786-0 ont été maintenues en culture dans un milieu RPMI 1640 (Invitrogen Sarl, Cergy Pontoise, France) contenant 300mg/l de L-Glutamine et supplémenté en NaHCO₃ (20mM), D-Glucose (25mM), tampon HEPES (10mM), pyruvate de sodium (1mM), de sérum foetal bovin (10% ; Invitrogen Sàrl) et d'un mélange d'antibiotiques (pénicilline 100U/ml et streptomycine 100 µg/ml) à 37°C dans un environnement contenant 10% de CO₂ dans l'air. Les cellules UOK-126 et UOK-128 ont été maintenues en culture dans un milieu DMEM (« Dulbecco Modified Eagle Medium ») contenant 10% de sérum foetal bovin et le mélange d'antibiotiques à 37°C dans un environnement contenant 10% de CO₂ dans l'air. Pour les expériences de prolifération, les cellules ont été sous-cultivées (passage) par digestion à la trypsine (Sigma, St. Quentin Fallavier, France) et traitées selon les conditions décrites ci-dessous.

### Animaux.

Les souris mâles athymiques nude Swiss Nu/Nu agées de 7 semaines à leur arrivée et pesant environ 30g ont été obtenues de Iffa-Credo (L'Arbresle, France). Les souris ont été hébergées dans des enceintes protégées et ventilées dans des conditions exemptes de pathogènes sous un cycle diurne 12h - nocturne 12h et avec libre accès à la nourriture et à l'eau de boisson préalablement autoclavées.

### Isolement des ARN et analyse de l'expression par RT-PCR.

L'ARN total est extrait des cellules tumorales en culture par la technique au Trizol (Invitrogen Sarl) selon le protocole défini par le fournisseur. L'obtention des ADNc totaux est réalisé par transcription réverse (RT) sur 2,5 à 10 µg d'ARN total dans un mélange contenant 200U de transcriptase réverse (Fermentas - Euromedex, Souffelweyerheim, France), 10U d'inhibiteur de RNase (Invitrogen Sàrl), 1mM de désoxynucléotides triphosphates (dNTP, Invitrogen Sàrl) et 1 µM d'amorce non spécifique p(dT)₁₅ (Roche Diagnostics, Meylan, France) pendant 1 h à 37°C. L'amplification par PCR est réalisée avec 0,15 µM d'amorces spécifiques :
- soit de la PTHrP humaine
   SEQ ID N°1 (sens): ATG CGA CGG AGA CTG GTT CAG;
   SEQ ID N°2 (antisens): TCA ATG CCT CCG TGA ATC GAG CTC CAG CGA CGT (fragment obtenu de 535 bp)
- soit du RPTH1 humain
   SEQ ID N°3 (sens): AGG AAC AGA TCT TCC TGC TGC A;
   SEQ ID N°4 (antisens): CAC AGC TAC GGT GAG GGA CGC CAG, (fragment obtenu de 515 bp)
- soit du VHL humain (Fukuzumi et al, Cancer Letters, 161 : 133-140, 2000)
   SEQ ID N°5 (sens): GCG TCG TGC TGC CCG TAT G ;
   SEQ ID N°6 (antisens) : TTC TGC ACA TTT GGG TGG TCT TC, (fragment obtenu de 340bp),
- soit du GAPDH humain
   SEQ ID N° 7 (sens) : GGA AGG TGA AGG TCG GAG TC.
   SEQ OD N° 8 (antisens) : GCA GTG ATG GCA TGG ACT G.
   0,4 mM de dNTP, 2,5 U de RedTaq DNA polymérase (Sigma) et 0,5 µg (PTHrP) ou 1 µg (RPTH1) d'ADNc. La PCR commence avec une dénaturation à 95°C pendant 4 min, puis les cycles sont programmés de la manière suivante : 1 min à 94°C (dénaturation), 1 min à 60°C (hybridation) et 1 min à 72°C (synthèse). La PCR est lancée pour 30 cycles pour la PTHrP et le RPTH1. Le dernier cycle est suivi d'une extension supplémentaire de 8 min à 72°C. Les produits de la PCR sont séparés par électrophorèse sur gel d'agarose à 2% en présence de 0.5 µg/ml de bromure d'éthidium. L'intensité des bandes obtenues après l'électrophorèse est quantifiée grâce à un logiciel d'analyse de gel (SigmaScanPro 4.01, Jandel Scientific, Erkrath, Germany).

### Dosage radioimmunologique (RIA) de la PTHrP

Les cellules tumorales ont été cultivées jusqu'à confluence dans les conditions normales de culture (milieu contenant 10% de sérum comme décrit ci-dessus). Les milieux de culture conditionnés des cellules ont alors été prélevés et congelés immédiatement à -80°C. Dans certaines expériences, les cellules ont été cultivées jusqu'à 80% de confluence, puis le milieu de culture a été changé pour du milieu frais. Les cellules ont alors été laissées encore 24h puis le milieu récupéré (dosage de la sécrétion de PTHrP en 24h). La PTHrP est alors dosée par une méthode radioimmunologique, utilisant un anticorps reconnaissant spécifiquement la partie amino-terminale de la PTHrP (kit RIA, Bachem).

### Western Blot

L'expression des protéines a été analysèe comme décrit dans Massfelder et al (2002, J. Am. Soc. Nephrol. 13, 639-648) sur 10-30 µg de protéines, en utilisant un anticorps monoclonal de souris anti-HA (Roche Diagnostics, Myelan, France) pour détecter pVHL ou un anticorps anti-hRPTH1. Un anticorps polyclonal de souris anti-β actine (Sigma-Aldrich) a été utilisé pour visualiser la charge en protéine du gel.

### Immunofluorescence de PTHrP et RPTH1.

Les cellules ont été préparées comme décrit dans Massfelder et al (1997, Proc. Natl. Acad. Sci. USA 94, 13630-13635). Des anticorps polyclonaux de lapin purifiés par affinité dirigés contre soit PTHrP(1-34) (N-term Ab) (Bachem), soit hRPTH1 (Eurogentec, Angers, France) ont été utilisés à 5 µg/ml. Un anticorps secondaire anti-lapin conjugué au TRITC a été utilisé pour la détection. Comme contrôle, un IgG de lapin non-immunisé (Sigma-Aldrich, St Quentin Fallavier, France) a été utilisé à la place de l'anticorps primaire.

### Plasmides et transfection stable.

***Vecteur d'expression***. Il a été utilisé le plasmide pCR3.1-Uni dans lequel l'ADNc complet du VHL humain (codant pour une protéine de 213 acides aminés) a été sous-cloné. Dans les transfections contrôles, nous avons utilisé le vecteur pCR3.1-Uni seul.

***Protocole de transfection***. Les cellules 786-0 ont été ensemencées dans des flacons de culture de 75cm² dans le milieu RPMI 1640 (cf. ci-dessus) supplémenté de 10% de sérum jusqu'à 50% de confluence. Les cellules adhérentes ont alors été lavées 3 fois avec 10 ml de milieu sans sérum puis maintenues dans 5 ml de ce même milieu. Pour transfecter les cellules, 5 µg d'ADN plasmidique (pCR3.1-Uni seul ou pCR3.1-Uni VHL) dans 50 µl d'eau sont incubés avec 50 µl de lipofectamine (Invitrogen Sarl) à 2 mg/ml pendant 30 min à température ambiante. Cette solution est ajoutée goutte à goutte aux cellules puis les boites incubées pendant 4 h à 37°C. Après cette période d'incubation 10 ml de milieu RPMI 1640 supplémenté de 10% de sérum ont été ajoutés aux cellules. Après 24h, le milieu est remplacé par du milieu frais contenant 10% de sérum. Deux jours après, le milieu est remplacé par du milieu supplémenté de 10% de FBS et contenant 500 µg/ml de G418 (néomycine) afin de sélectionner les cellules transfectées. La concentration optimale de G418 a été déterminée pour les cellules 786-0 par une « courbe de mort » sur 3 semaines avec des concentrations de 25 à 1000 µg/ml de G418 et changement de milieu tous les 3 jours. Pour chaque transfection (vecteur seul ou pCR3.1-Uni VHL), 3 clones ont été sélectionnés ainsi que l'ensemble des clones (tous les clones rassemblés) et maintenus dans le milieu RPMI 1640 avec sérum et supplémenté de 500 µg/ml de G418.

### Mesure de la prolifération cellulaire.

### Traitements des cellules.

Les cellules ont été mises en culture dans des boîtes à 24 puits (1 ml par puit) et dans des boîtes à 96 puits (100 µl par puit) à raison de 10 000 à 30 000C/ml. Les cellules ont été cultivées dans le milieu avec le sérum décrit ci-dessus. Pour les expériences avec l'antagoniste des RPTH1, après 24 h de culture le milieu a été changé pour un milieu sans sérum contenant 0,1 % de BSA (albumine sérique bovine, Sigma) et les antibiotiques. Cette manipulation permet de cultiver les cellules en absence des facteurs présents dans le sérum et permet d'apprécier l'influence de l'antagoniste sur la prolifération cellulaire. Après 24h, les milieux de culture sont remplacés par un milieu toujours sans sérum mais contenant l'antagoniste (Asn10, Leu11, D-Trp12)PTHrP(7-34) amide à diverses concentrations soit le diluant (contrôle) pendant 48h. Pour les expériences avec les anticorps anti-PTHrP, le milieu de culture avec sérum est maintenu et après 24h de culture les cellules sont cultivées en présence soit des anticorps anti-PTHrP (anti-PTHrP(1-34) à 1,5µg/ml, anti-PTHrP(34-53) à 2µg/ml ou anti-PTHrP(107-139) à 5µg/ml soit d'IgG purifiés (5µg/ml, Sigma) pendant encore 48h.

La prolifération cellulaire a été déterminée par deux moyens, d'une part en comptant les cellules (comptage manuel) à partir des boîtes à 24 puits et d'autre part en mesurant l'incorporation de 5-bromo-2'-déoxyuridine (BrdU) à partir des boîtes à 96 puits.

### Comptage des cellules

Le milieu de culture est retiré par aspiration et un volume adéquat de trypsine-EDTA (Sigma) est ajouté. Les boîtes à 24 puits sont alors mises à l'étuve à 37°C pendant 5 à 10 min jusqu'à ce que les cellules soient bien décollées du fond des boîtes et en suspension individuelle, ce qui est suivi sous microscope optique. Les cellules sont alors récupérées et comptées manuellement à l'aide d'une cellule de comptage placée sous un microscope optique. Un puit est utilisé par condition expérimentale.

### Mesure de l'incorporation de BrdU

Le principe du test utilisé est le suivant: le BrdU, un analogue des pyrimidines, est incorporé à la place de la thymine dans l'ADN des cellules en prolifération. La mesure de l'incorporation du BrdU est un indice de la synthèse d'ADN. Le test est effectué grâce à un kit de réaction colorimétrique disponible dans le commerce (Roche Diagnostics) et selon le protocole décrit par le fournisseur. La couleur développée et de ce fait les valeurs d'absorbance lues dans le visible sont directement corrélées au taux de synthèse d'ADN et ainsi au nombre de cellules en prolifération dans les différentes cultures.

### Analyse de la mort cellulaire/apoptose

Les cellules ont été traitées dans des conditions contrôles ou avec soit un anticorps anti-PTHrP N-terminal à 1,5 µg/ml, soit un antagoniste de RPTH1 à 1 µM pendant 48 h. L'acridine orange (AO) et le bromure d'éthidium (EB) ont été dissous dans un tampon PBS et ajoutés à 2 µg/ml dans le milieu de culture. Les cellules ont été observées et photographiées sous microscopie à fluorescence, soit pour le FITC (AO) soit pour le TRITC (EB). La fragmentation de l'ADN internucléosomal a été détectée par un test « DNA laddering ». Les cellules ont été resuspendues dans 300 µl de tampon de lyse (25 mM EDTA, 1 % SDS, 1 mg/ml de protéinase K, pH 8) et incubées la nuit à 50 °C. L'ADN a été extrait par phénol/chloroforme, précipité à l'éthanol et analysé par électrophorèse sur gel d'agarose.

### Mesure de l'activité du promoteur de PTHrP

Les cellules 786-0 non transfectées, et les clones transfectés par le vecteur ou le vecteur-VHL ont été transfectés transitoirement avec une construction contenant les régions promotrices (Promoteurs P1, P2, et P3) du gène PTHrP liées à un gène chloramphenicol acétyltransférase (CAT) bactérien dénué de promoteur. La transfection a été effectuée 24 h après le repiquage des cellules avec 1 µg/cm² de plasmide promoteur PTHrP-CAT ou un plasmide CAT dénué de promoteur en présence de Lipofectamine. Les cellules ont été analysées 48 h après la transfection pour l'expression de CAT en utilisant le kit ELISA pour l'enzyme CAT (Roche Diagnostic). Les résultats ont été normalisés et exprimés en pg d'enzyme CAT/ mg de protéine.

### Tests Actonomycin D

Les cellules 786-0 non transfectées, et les clones transfectés par le vecteur ou le vecteur-VHL ont été exposés à 5 µg/ml d'actinomycine D (Sigma) pendant 0-30 h. Les ARN totaux ont été isolés et l'expression de hPTHrP a été analysée par RT-PCR en utilisant hGAPDH pour normaliser les réactions de PCR.

### Protocoles expérimentaux chez la souris nude.

***Implantation sous-cutanée des cellules 786-0**.* Les cellules 786-0 ont la particularité d'être tumorigéniques *in vivo* chez la souris nude et sont donc couramment utilisées pour étudier l'effet des drogues sur la croissance tumorale humaine sur ce modèle.

Les cellules 786-0 ont été cultivées dans le milieu RPMI 1640 supplémenté de sérum jusqu'à confluence. Les cellules ont été récupérées par trypsination et resuspendu dans du milieu RPMI 1640 sans sérum et sans antibiotiques à raison de 5 millions de cellules/200µl le jour même de l'implantation des souris. Les cellules ont été implantées en sous-cutané au niveau du dos chez les souris nude à raison de 200µl par implantation. Une semaine à 10 jours après l'implantation des cellules les tumeurs se sont développées formant des nodules d'environ 60 à 300 mm³. A partir de ce moment les traitements ont été initiés selon trois protocoles : un protocole pilote qui a été réalisé afin de déterminer l'efficacité des traitements *in vivo* et réalisé sur deux souris par condition expérimentale recevant 4 implantations (soit 8 tumeurs/condition expérimentale), et deux protocoles expérimentaux élaborés suite aux résultats obtenus avec le protocole pilote sur 10 ou 5 souris par condition expérimentale et recevant 1 ou 2 implantations (soit 10 tumeurs/condition expérimentale).

***Protocole pilote**.* Dans ce protocole, 6 souris ont été séparées en trois groupes : un premier groupe recevant des injections d'eau stérile (contrôle), un deuxième recevant des injections d'anticorps anti-PTHrP (1-34) (Bachem, dissout dans l'eau stérile) et un troisième recevant des injections d'antagoniste (Asn10, Leu11, D-Trp12)PTHrP(7-34) amide (Bachem, dissout dans l'eau stérile). L'ensemble des injections a été réalisé 2 fois par semaine en intra-péritonéal (50µg/souris d'anticorps ou 20µg/souris d'antagoniste dans 200µl) et en intra-tumoral (25µg/souris d'anticorps ou 10µg/souris d'antagoniste dans 100µl). Les souris contrôle recevant une injection d'eau de 200µl en intra-péritonéal et de 100µl d'eau en intra-tumoral. Le volume des tumeurs (longueur x largeur x profondeur x 0,5236) a été mesuré de manière aveugle à l'aide d'un pied à coulisse manuellement aux jours 0, 4, 7, 12, 14, 18, 23 et 25 après la première injection des substances sous anesthésie gazeuse légère à l'isoflurane. Pour la série « anticorps », les injections ont été stoppées après 18 jours.

**Premier protocole expérimental.** Les résultats de la série pilote ont montré que les anticorps anti-PTHrP et l'antagoniste des RPTH1 ont une efficacité égale à inhiber la croissance tumorale des tumeurs implantées chez la souris nude. Par conséquent, dans ce protocole d'une part l'effectif de chaque groupe a été augmenté (10 souris avec une tumeur / groupe expérimental) et d'autre part des injections quotidiennes soit d'eau (contrôle) soit de l'antagoniste des RPTH1 (dissout dans l'eau stérile) ont été réalisées. Comme pour le protocole pilote, l'ensemble des injections a été réalisé en intra-péritonéal (200µl d'eau ou 20µg/souris d'antagoniste dans 200µl) et en intra-tumoral (100µl d'eau ou 10µg/souris d'antagoniste dans 100µl). Pour cette série, la taille des tumeurs a été mesurée de manière aveugle deux fois par semaine sous anesthésie gazeuse légère à l'isoflurane. A l'issue de la période expérimentale les souris ont été anesthésiées au pentobarbital. Environ 0,5 ml de sang a alors été récupéré rapidement par ponction intracardiaque de chaque souris pour le dosage des électrolytes et de la PTHrP circulante. Les tumeurs ont été disséquées et fixées dans le formol pour les études histo-pathologiques (aspect nécrotique) et immunohistochimiques ultérieures (facteur VIII, PTHrP, RPTH1, index mitotique et index apoptotique).

**Deuxième protocole expérimental.** Parallèlement, un protocole expérimental a également été réalisé pour les anticorps anti- PTHrP (N-term) avec des injections quotidiennes intra-péritonéal avec 40 µg/souris soit d'anticorps anti-PTHrP (N-term) soit d'IgG non-spécifique (5 souris avec 2 tumeurs / groupe expérimental).

**Index prolifératif et apoptotic.** L'index de prolifération a été déterminé par coloration de coupe de tumeur avec un anticorps de souris monoclonal anti-Ki67 humain Dako, Trappes, France) en utilisant des méthodes standard. Le kit de détection de cellules apoptotiques, basé sur la méthode de TUNEL (Roche Diagnostics) a été utilisé pour mesurer l'index apoptotique sur les coupes. Les cellules totales et colorées dans 15 champs (0,25 mm² chacun) de coloration cellulaire la plus élevée ont été comptées pour déterminer les deux index, qui sont exprimés en pourcentage de cellules colorées par rapport aux cellules totales.

**Facteur VIII et densité microvasculaire.** Les coupes de tumeur ont été colorées pour les cellules endothéliales avec un anticorps polyclonal de lapin anti-facteur VIII humain (Dako) en utilisant les méthodes standards. La densité des microvaisseaux a été déterminée par comptage des points d'intersection entre vaisseaux et du nombre total de vaisseaux de 4 à 5 champs (0,25 mm² chacun) montrant la plus haute densité vasculaire.

### Statistiques

Pour les expériences *in vitro*, les résultats des comptages manuels sont exprimés en pourcentage par rapport au nombre de cellules présentes dans les puits contrôles. Pour calculer les incorporations de BrdU en réponse aux traitements, l'ensemble des valeurs de DO obtenues sont diminuées de la moyenne des DO obtenues pour les puits contrôles négatifs. Les incorporations en réponse aux traitements sont alors exprimées en pourcentage par rapport aux incorporations des contrôles positifs prises comme 100 % d'incorporation. Les résultats sont exprimés en moyenne +/- ESM et ont été comparés par test-t ou test-t apparié. Une valeur de P au moins < 0.05 est considérée comme reflétant une différence significative.

Pour les expériences *in vivo* chez la souris nude, les résultats de croissances tumorales ont été exprimés en pourcentage de la taille au jour 0 des traitements. Les résultats sont exprimés en moyenne +/- ESM. La croissance des tumeurs selon les traitements a été comparée par test-t pour évaluer les différences entre les traitements et par test-t apparié pour apprécier l'évolution de la croissance tumorale dans chaque condition expérimentale. Une valeur de P au moins <0.05 est considérée comme reflétant une différence significative.

### Résultats

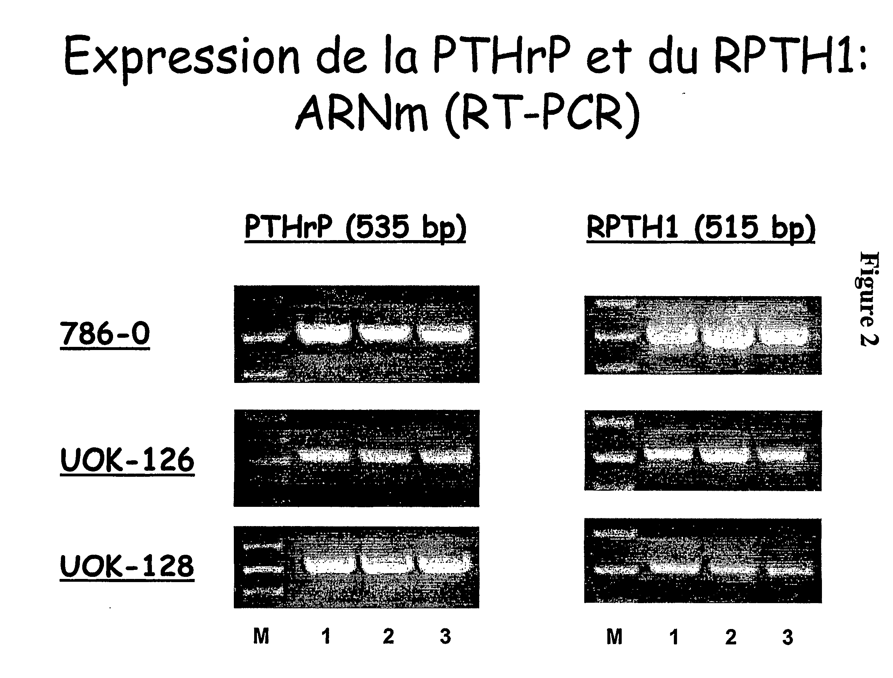

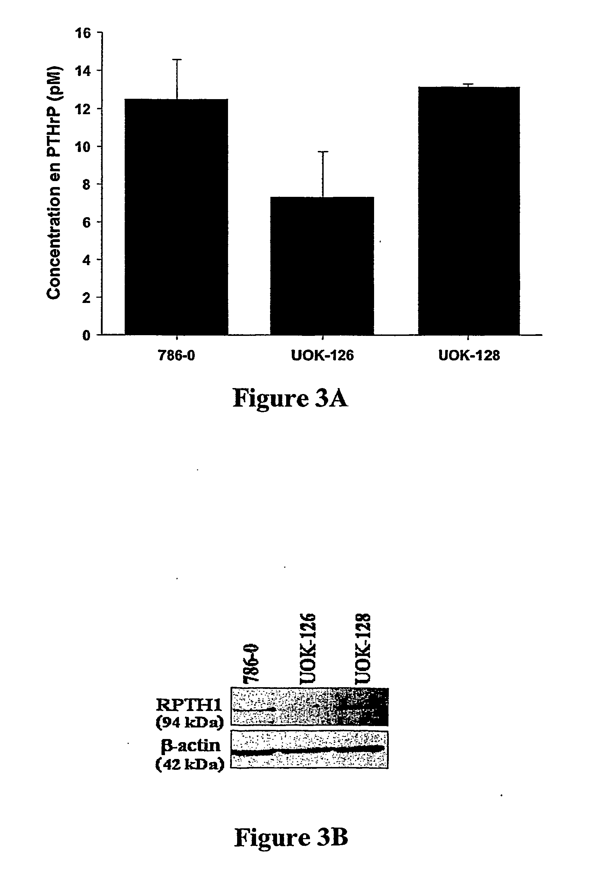

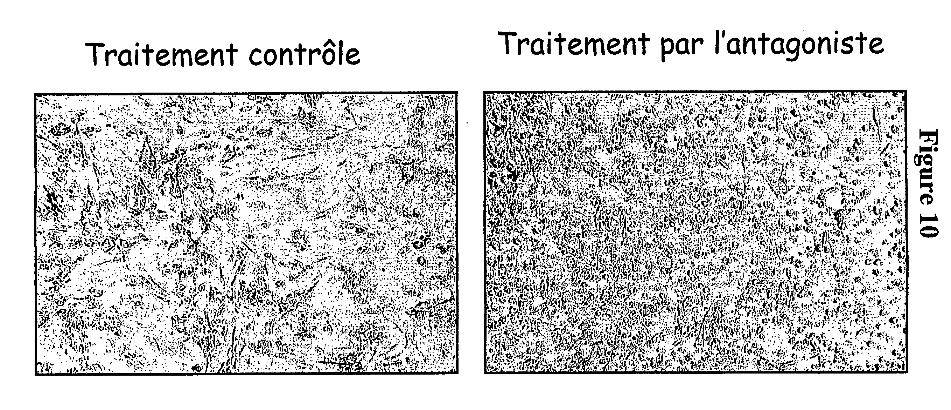

- Les gels montrant l'expression de la PTHrP et du RPTH1 au niveau de l'ARNm dans 3 échantillons (numéroté 1, 2 et 3) des 3 lignées tumorales 786-0, UOK-126 et UOK-128 sont donnés à la figure 2. Expériences réalisées en RT-PCR. On constate aisément que les trois lignées expriment la PTHrP et le RPTH1.
- Les résultats représentés sur la figure 3 montrent que les 3 lignées tumorales expriment la PTHrP à l'échelle de la protéine. A titre de comparaison, la sécrétion de PTHrP immunoréactive est indétectable dans les cellules musculaires lisses vasculaires par exemple alors que l'expression de la PTHrP est observable par RT-PCR (résultats non montrés). Avec les résultats obtenus en RT-PCR, on en conclut que les 3 lignées expriment la PTHrP au niveau du messager et au niveau de la protéine, ce qui permet d'envisager des effets autocrines/paracrines de la PTHrP sur la prolifération des cellules.
   Dans des cellules en culture, PTHrP et RPTH1 ont été localisés par immunofluorescence dans le cytosol et dans les nucléoles. L'allure du marquage était identique pour les trois types cellulaires.

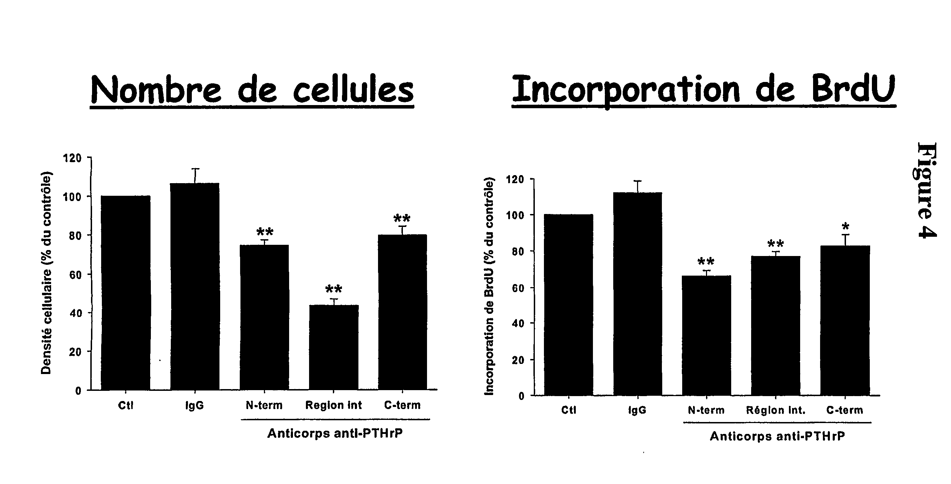

- La figure 4 représente l'effet des anticorps dirigés contre les diverses régions de la PTHrP sur la prolifération des cellules tumorales 786-0 *in vitro* mesurée sur le nombre de cellules (à gauche) et sur l'incorporation de BrdU (à droite). La figure 4 montre que les divers anticorps inhibent de 20 à 60 % la prolifération cellulaire, suggérant un effet auto/paracrine de la PTHrP sécrétée sur la prolifération des cellules.
   Le fait que les trois anticorps inhibent la prolifération de ces cellules suggèrent que ces cellules sécrètent entre autres des gros fragments amino-terminaux reconnus par les trois anticorps.
   N-term: anticorps anti-PTHrP(1-34) (Bachem) 1,5 µg/ml
   Région int.: anticorps anti-PTHrP (34-53) (Ab-2, Oncogene) 2 µg/ml
   C-term: anticorps anti-PTHrP(107-139) (P. Esbrit, Madrid, Espagne) 5 µg/ml
   IgG: (Sigma) 5 µg/ml
   Nombre d'expériences indépendantes: n=8-12 (figure 4 de gauche); n=4 (figure 4 de droite).

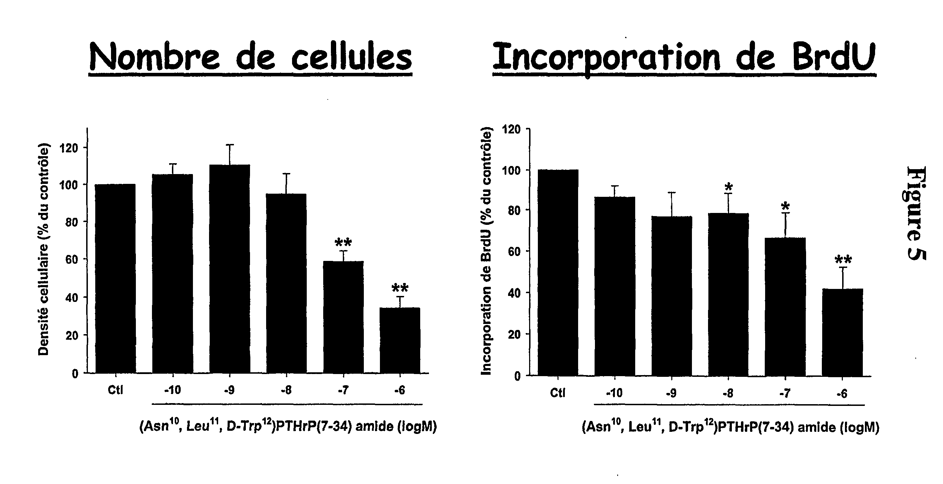

- La figure 5 représente l'effet de l'antagoniste des RPTH1, la (Asn10, Leu11, D-Trp12)hPTHrP(7-34) amide à diverses concentrations sur la prolifération des cellules tumorales 786-0 *in vitro* mesurée sur le nombre de cellules (à gauche) et sur l'incorporation de BrdU (à droite). Ces résultats montrent clairement la capacité de l'antagoniste à inhiber très fortement à 70% la prolifération cellulaire. Nombre d'expériences indépendantes: n=12 (figure 5 de gauche); n=7-15 (figure 5 de droite).

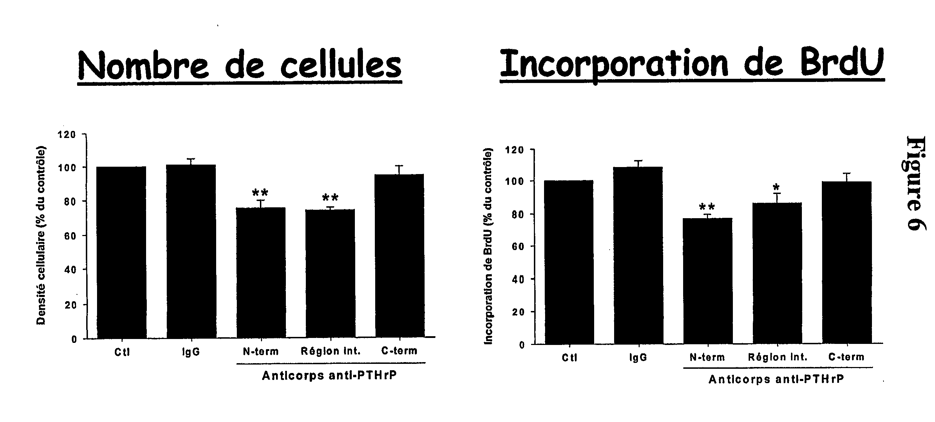

- La figure 6 représente l'effet des anticorps dirigés contre les diverses régions de la PTHrP sur la prolifération des cellules tumorales UOK-126 *in vitro* mesurée sur le nombre de cellules (à gauche) et sur l'incorporation de BrdU (à droite). La figure montre que les divers anticorps inhibent de 20 à 30 % la prolifération cellulaire, suggérant un effet auto/paracrine de la PTHrP sécrétée sur la prolifération des cellules. Seul l'anticorps dirigé contre la partie C-term ne montre pas d'efficacité. Cela peut être lié soit à une dilution trop forte de l'anticorps soit au fait que ces cellules sécrètent des fragments amino-terminaux de la PTHrP mais ne présentant pas une immunoréactivité C-terminale.Nombre d'expériences indépendantes: n=4-12 (figure 6 de gauche); n=4-12 (figure 6 de droite).

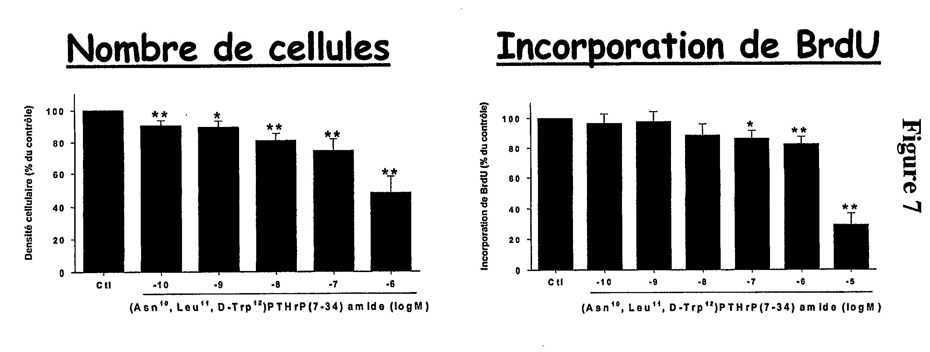

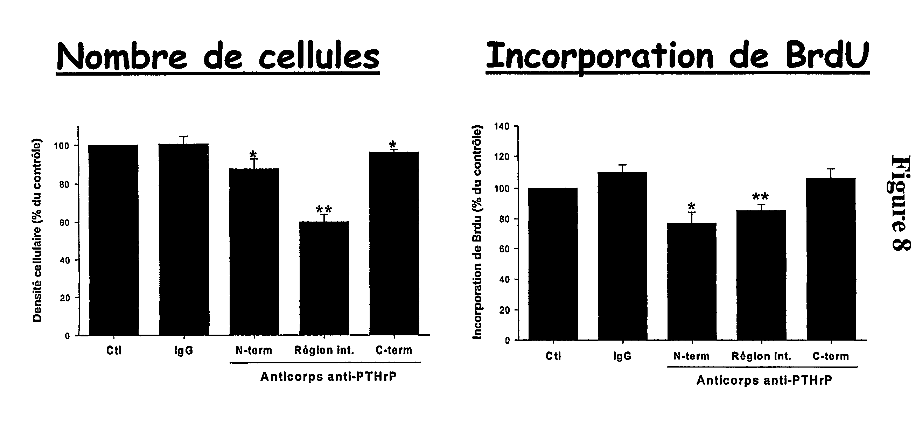

- La figure 7 représente l'effet de l'antagoniste des RPTH1, la (Asn10, Leu11, D-Trp12)hPTHrP(7-34) amide à diverses concentrations sur la prolifération des cellules tumorales UOK-126 *in vitro* mesurée sur le nombre de cellules (à gauche) et sur l'incorporation de BrdU (à droite). Ces résultats montrent clairement la capacité de l'antagoniste à inhiber très fortement là aussi la prolifération cellulaire. Nombre d'expériences indépendantes: n=8 (figure 7 de gauche); n=8 (figure 8 de droite).
- La figure 8 représente l'effet des anticorps dirigés contre les diverses régions de la PTHrP sur la prolifération des cellules tumorales UOK-128 *in vitro* mesurée sur le nombre de cellules (à gauche) et sur l'incorporation de BrdU (à droite). La figure montre que les divers anticorps inhibent de 20 à 40 % la prolifération cellulaire, suggérant un effet auto/paracrine de la PTHrP sécrétée sur la prolifération des cellules. Comme pour les cellules UOK-126, seul l'anticorps dirigé contre la partie C-term montre une efficacité limitée. Avec les résultats obtenus sur la lignée UOK-126, ces résultats suggèrent que cette faiblesse d'efficacité soit due à une dilution trop forte de l'anticorps. Cependant, cet anticorps n'a pas été en quantité suffisante pour augmenter la concentration de l'anticorps dans le milieu.Nombre d'expériences indépendantes: n=4-12 (figure 8 de gauche); n=4-12 (figure 8 de droite).

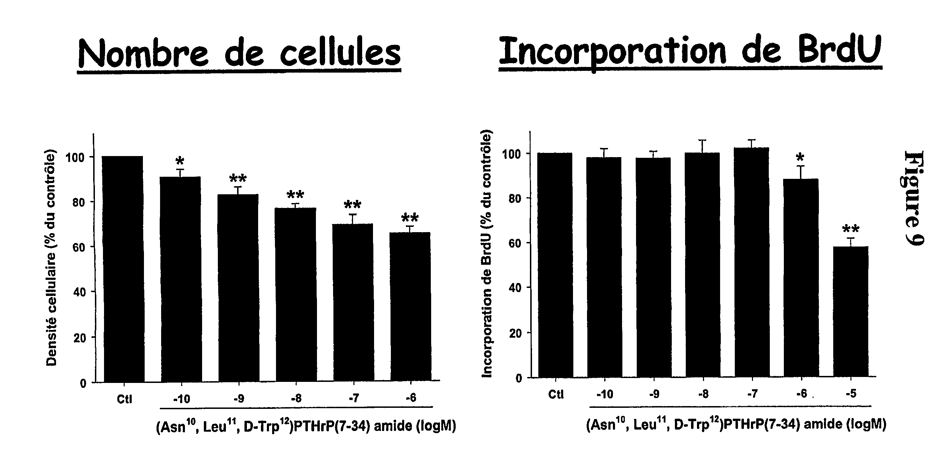

- La figure 9 représente l'effet de l'antagoniste des RPTH1, la (Asn10, Leu11, D-Trp12)hPTHrP(7-34) amide à diverses concentrations sur la prolifération des cellules tumorales UOK-128 *in vitro* mesurée sur le nombre de cellules (à gauche) et sur l'incorporation de BrdU (à droite). Ces résultats montrent clairement la capacité de l'antagoniste à inhiber très fortement la aussi la prolifération cellulaire. Nombre d'expériences indépendantes: n=8 (figure de gauche); n=8 (figure de droite)
   Dans les figures 4 à 9 :
   * signifie p<0,05 par rapport au contrôle
   ** signifie p<0,01 par rapport au contrôle
- La figure 10 montre des photographies des cellules 786-0 en culture dans un milieu RPMI 1640 sans sérum (avec 0,1% de BSA) et traitées soit en contrôle (à gauche) soit en présence de 10-6 M de l'antagoniste (Asn10, Leu11, D-Trp12)hPTHrP(7-34) amide (à droite) pendant 48h.
   On observe clairement que l'antagoniste a comme effet de détacher les cellules du support de culture leur faisant perdre complètement leur adhérence.
   Cet effet peut être dû à un effet toxique et/ou un effet apoptotique et/ou un effet nécrotique et/ou un effet anoikitique (apoptose cellulaire par détachement des cellules).
- La figure 11 représente l'effet de l'antagoniste des RPTH1, la (Asn10, Leu11, D-Trp12)hPTHrP(7-34) amide et d'un anticorps dirigé contre l'extrémité N-terminale de PTHrP (N-term Ab) sur l'apoptose des cellules tumorales 786-0. Pour déterminer si l'inactivation de PTHrpP/RPTH1 induit la mort cellulaire, les inventeurs ont examiné simultanément des coloration AO et EB pour un contrôle et pour les cellules CCR traitées. EB imprègne les cellules vivantes, agissant uniquement en marqueur de la mort cellulaire, alors que AO imprègne librement et visualise toutes les cellules du champ. L'exposition à un antagoniste de RPTH1 augmente nettement le nombre de cellule marquée par EB comparé aux cellules non-traitées (Fig. 11A). L'analyse de l'ADN génomique dans les cellules 786-0 traitées a révèlé une fragmentation de l'ADN internucléosomal (échelle à 180 pb) spécifique de la mort cellulaire induite par apoptose (Fig. 11 B). Le détachement cellulaire, la coloration ainsi que la fragmentation de l'ADN internucléosomal ont été observés pour toutes les lignées cellulaires et quelque soit le moyen utilisé pour bloquer le système PTHrP/RPTH1. Ces résultats suggèrent fortement que PTHrP agit comme un facteur essentiel de croissance et de survie pour les cellules CCR.
- La figure 12 représente la croissance des tumeurs des souris traitées soit en contrôle soit par l'anticorps soit par l'antagoniste selon le protocole pilote décrit ci-dessus. Les résultats de croissance sont exprimés en pourcentage par rapport au jour 0 du début des traitements.
   On constate aisément que les tumeurs contrôle croissent de manière très importante alors que les tumeurs des souris traitées soit par l'anticorps anti-PTHrP(1-34) soit par l'antagoniste ont une croissance considérablement ralentie par rapport à la croissance des tumeurs des souris traitées en contrôles.
   Les photographies montrent en haut une souris traitée en contrôle pendant 25 jours avec ses 4 tumeurs bien visibles et en bas une souris traitée pendant 25 jours par l'antagoniste. L'efficacité de l'antagoniste à inhiber la croissance tumorale est évidente.
   Nombre d'expériences indépendantes: n=8 (4 tumeurs/souris, 2 souris/conditions expérimentales)
   * signifie p<0,05 pour la croissance des tumeurs des souris traitées par l'anticorps ou l'antagoniste par rapport à la croissance des tumeurs des souris traitées en contrôle au même jour
   ** signifie p<0,01 pour la croissance des tumeurs des souris traitées par l'anticorps ou l'antagoniste par rapport à la croissance des tumeurs des souris traitées en contrôle au même jour
   # signifie p<0,01 pour la croissance des tumeurs des souris traitées en contrôle par rapport au jour 0 de traitement (croissance significative à partir du jour 7)
   ## signifie p<0,01 pour la croissance des tumeurs des souris traitées par l'anticorps ou l'antagoniste par rapport au jour 0 (croissance significative à partir du jour 12 seulement)

   Des études statistiques, il apparaît que le traitement deux fois par semaine par l'anticorps ou l'antagoniste des souris est suffisant pour ralentir fortement la croissance tumorale par rapport à la croissance contrôle mais pas pour inhiber complètement cette croissance. C'est pour cela que dans la série expérimentale, il a été décidé d'injecter l'antagoniste tous les jours afin d'évaluer si l'antagoniste est capable d'inhiber complètement la croissance tumorale, voire de la faire régresser. Dans la série expérimentale, le nombre n de souris a été augmenté à 10 par conditions expérimentales.
- La figure 13 représente la croissance des tumeurs des souris traitées soit en contrôle soit par l'antagoniste selon le protocole expérimental décrit ci-dessus. Les résultats de croissance sont exprimés en pourcentage par rapport au jour 0 du début des traitements.
   On constate à nouveau que les tumeurs contrôle croissent de manière très importante alors que les tumeurs des souris traitées par l'antagoniste ont une croissance cette fois inhibée totalement (donc bloquée) par rapport à la croissance des tumeurs des souris traitées en contrôles. Cette étude révèle que l'antagoniste exerce au moins des effets cyto-statiques sur les tumeurs.
   Nombre d'expériences indépendantes : n=10 souris (1 tumeur/souris) par conditions expérimentales
   * signifie p<0,05 pour la croissance des tumeurs des souris traitées par l'antagoniste par rapport à la croissance des tumeurs des souris traitées en contrôle au même jour
   ** signifie p<0,01 pour la croissance des tumeurs des souris traitées par l'antagoniste par rapport à la croissance des tumeurs des souris traitées en contrôle au même jour
   # signifie p<0,05 pour la croissance des tumeurs des souris traitées en contrôle par rapport au jour 0 de traitement (croissance significative dès le jour 3)
   ## signifie p<0,01 pour la croissance des tumeurs des souris traitées en contrôle par rapport au jour 0 de traitement

   Des études statistiques, il apparaît cette fois que le traitement tous les jours des souris par l'antagoniste bloque complètement la croissance tumorale. Certaines tumeurs ont même régressé.
   La figure 14 représente la croissance des tumeurs des souris traitées soit par un anticorps dirigé contre l'extrémité N-terminale de PTHrP soit par des IgG non-spécifiques selon le protocole expérimental décrit ci-dessus. Les résultats de croissance sont exprimés en pourcentage par rapport au jour 0 du début des traitements. De manière surprenante et impressionante, 7 tumeurs ont disparu, alors que les 3 autres ont régressé de 50 à 80 % dans les souris traitées par les anticorps par rapport aux contrôles.
   Nombre d'expériences indépendantes : n=10 tumeurs (2 tumeurs/souris) par conditions expérimentales
   ## signifie p<0,01 pour la croissance des tumeurs des souris traitées en contrôle par rapport au jour 0 de traitement
   ** signifie p<0,01 pour la croissance des tumeurs des souris traitées par l'anticorps par rapport à la croissance des tumeurs des souris traitées en contrôle au même jour

   Puisque 70 % des tumeurs disparaissent avec le traitement par un anticorps anti-PTHrP et que les tumeurs restantes sont trop petites, les analyses histopathologiques ont été réalisées sur les tumeurs des animaux traités par un antagoniste de RPTH1 et leur contrôle. Auncune différence polymorphique n'a été détectée entre les deux groupes. Les tumeurs obtenues des souris contrôles expriment fortement PTHrP et RPTH1 avec une coloration claire observée dans le cytoplasme de toutes les cellules et dans le nucléole de cellules variées. L'index de prolifération n'était pas différents entre les groupes contrôles et traitées. Par contre, le traitement par l'antagoniste de RPTH1 a doublé l'index apoptotique. De plus, le nombre de points d'intersection des vaisseaux et le nombre total de vaisseaux par aire de surface ont été significativement augmentés.
   Globalement, le système ciblant PTHrP/RPTH1 est non seulement efficace contre CCR par une augmentation de l'apoptose des cellules tumorales mais également apparaît ne pas avoir d'effets secondaires nuisibles.
- Sur la figure 15 sont représentés à gauche les vecteurs pCR3.1-Uni seul ou codant pour le VHL(1-213). Ces vecteurs ont été transfectés dans les cellules 786-0 (cf. ci-dessus). Trois clones individuels ont été isolés après sélection au G418 ainsi que l'ensemble des clones pour les cellules transfectées par le vecteur seul (786-0 V, clones individuels 2, 3, 4 et clones mélangés p) ou par le vecteur codant pour le VHL (786-0 VHL, clones individuels 2, 3 et 6 et clones mélangés p). Parallèlement à ces clones, 3 échantillons de cellules 786-0 non transfectées (786-0 wt pour « wild-type ») et 3 échantillons de cellules HK-2 (cellules tubulaires proximales humaines normales) ont également été testées pour l'expression du VHL et de la PTHrP.
   Les gels représentent l'expression du VHL obtenue par RT-PCR sur l'ARN total isolé des diverses cellules (bande à 340bp) selon le protocole décrit ci-dessus. On constate aisément que les cellules HK-2 normales expriment 2 fois plus de VHL que les cellules 786-0 wt (1 allèle du gène VHL absent). Dans les clones transfectés avec le vecteur seul l'expression du VHL est équivalente à ce qui est retrouvée dans les cellules 786-0 non transfectées. Par contre, dans les clones transfectés par le VHL, l'expression du VHL est bien augmentée ce qui montre que la transfection est très efficace. Si l'on regarde maintenant l'expression de la PTHrP dans les divers clones et types cellulaires, on observe facilement une corrélation inverse entre l'expression du VHL et l'expression de la PTHrP. Ces résultats montrent que la PTHrP au niveau de son messager est une cible directe ou indirecte du gène suppresseur de tumeurs VHL. Afin de confirmer cela au niveau de la protéine elle-même (PTHrP immunoréactive), les milieux conditionnés des 786-0 wt, V et VHL ont été récupérés. Les résultats sont représentés sur la figure 17. De plus, les Western Blots de la Figure 16 permettent d'estimer les niveaux de protéine pour VHL.
- La Figure 17 de gauche montre par RIA de la PTHrP, dans les milieux conditionnés de cellules à confluence, que la production de PTHrP est diminuée de 50 % dans les cellules 786-0 transfectées avec le plasmide codant pour le VHL (786-0 VHL) par comparaison aux cellules non transfectées (786-0 wt) ou transfectées avec le vecteur seul (786-0 V).
   Afin de confirmer ce résultat, les cellules ont été cultivées jusqu'à 80% de confluence puis le milieu changé pour du milieu frais et laisser 24h afin de mesurer la sécrétion de PTHrP en 24h. Les cellules ont parallèlement été comptées afin d'exprimer la sécrétion de PTHrP par 24h et par million de cellules. Les résultats de ces expériences sont représentés sur la figure 17 de droite représentant la sécrétion de PTHrP en pM par 24h et par million de cellules. Là encore, on constate aisément que la sécrétion de PTHrP est diminuée de 50% dans les cellules 786-0 VHL par rapport aux cellules contrôles non transfectées ou transfectées par le vecteur seul. D'autre part, l'expression de RPTH1 n'est pas affectée par pVHL. Ainsi, PTHrP est réprimé par pVHL. Ces résultats montrent avec ceux présentés sur la figure 15 que la PTHrP est une cible directe ou indirecte (par exemple via le HIF pour « hypoxic-induced factor ») du produit du gène VHL dans les cellules tumorales.
   Nombre d'expériences indépendantes: n=12-14 (figure de gauche) et n=5 (figure de droite)
   * Signifie p<0,01 pour la sécrétion de PTHrP des cellules 786-0 VHL par rapport aux cellules contrôles 786-0 wt et 786-0 V

Pour analyser si PTHrP est régulé par pVHL au niveau transcriptionnel, les inventeurs ont transfecté de manière transitoire les cellules 786-0 non-transfectées, les clones 3 et 4 de cellules 786-0 transfectées par le vecteur seul, les clones 2 et 3 de cellules 786-0 transfectées par le vecteur codant pour le VHL avec un vecteur d'expression de CAT contenant les régions promotrices du gène PTHrP humain et ils ont mesuré l'enzyme CAT dans les extraits cellulaires. La réintroduction du gène VHL dans les cellules 786-0 n'altère pas l'activité transcriptionnelle des promoteurs PTHrP (Figure 18) suggérant que la régulation est post-transcriptionnelle. L'analyse de la stabilité des ARNm de PTHrP dans les cellules 786-0 non-transfectées, les clones 3 et 4 de cellules 786-0 transfectées par le vecteur seul, les clones 2 et 3 de cellules 786-0 transfectées par le vecteur codant pour le VHL soumis à un inhibiteur transcriptionnel, actinomycin D, montre en effet que la demie-vie des ARNm de PTHrP était de 38,3 ± 3 h et 40,1 ± 3,4 h pour les cellules 786-0 non-transfectées et les cellules 786-0 transfectées par le vecteur seul, respectivement, et est diminuée de 50 % à 20,9 ± 2,6 h pour les cellules 786-0 transfectées par le vecteur codant pour le VHL (Figure 19). Ansi, pVHL diminue l'expression de PTHrP dans le CCR en déstabilisant les ARNm de PTHrP.

Ce lien entre la PTHrP et le VHL suggère fortement que la PTHrP puisse être impliquée dans la croissance de la majorité des CCC, et de ce fait que l'utilisation des anticorps anti-PTHrP ou des antagonistes des RPTH1 constitue un outil thérapeutique chez l'homme très prometteur pour lutter contre ce fléau. L'ensemble des résultats présentés ici *in vitro* et *in vivo* vont largement dans ce sens.

## Revendications

1. Utilisation d'au moins un antagoniste de la PTHrP dans la préparation d'une composition pharmaceutique destinée à traiter, de manière préventive ou curative, un cancer du rein et en particulier le carcinome à cellules rénales (CCR) chez le mammifère et en particulier chez l'homme, ledit antagoniste étant un anticorps anti-PTHrP(34-53).

2. Utilisation selon la revendication précédente, **caractérisée en ce que** la composition est destinée à traiter le carcinome papillaire (chromophiles), le carcinome à cellules chromophobes, le carcinome de Bellini ou les carcinomes à cellules rénales non classées, ou de préférence le carcinome à cellules claires (CCC).

3. Utilisation selon l'une des revendications précédentes, **caractérisée en ce que** la composition est destinée à inhiber ou diminuer la croissance tumorale et/ou la formation de métastases dans le cancer du rein et/ou ses évolutions métastatiques, en particulier au niveau du poumon et du foie.

4. Utilisation selon l'une des revendications précédentes, **caractérisée en ce que** la composition est destinée au traitement de tumeurs malignes du cancer du rein, plus spécifiquement au traitement de tumeurs malignes solides.

5. Utilisation selon l'une des revendications 1 à 4, **caractérisée en ce que** l'anticorps anti-PTHrP est choisi parmi un anticorps humanisé, un anticorps humain, un anticorps chimérique, un anticorps obtenu à partir d'un hybridome ou un fragment de ces anticorps et/ou une forme modifiée dudit fragment.

6. Utilisation selon la revendication 5, **caractérisée en ce que** l'anticorps anti-PTHrP est un anticorps monoclonal.

7. Antagoniste de la PTHrP pour le traitement, préventif ou curatif, d'un cancer du rein, et en particulier le carcinome à cellules rénales (CCR), chez le mammifère, et en particulier chez l'homme, ledit antagoniste étant étant un anticorps anti-PTHrP(34-53).

8. Antagoniste de la PTHrP selon la revendication 7, **caractérisé en ce que** la composition est destinée à traiter le carcinome papillaire (chromophiles), le carcinome à cellules chromophobes, le carcinome de Bellini ou les carcinomes à cellules rénales non classées, ou de préférence le carcinome à cellules claires (CCC).

9. Antagoniste de la PTHrP selon l'une des revendications 7-8, **caractérisé en ce que** la composition est destinée à inhiber ou diminuer la croissance tumorale et/ou la formation de métastases dans le cancer du rein et/ou ses évolutions métastatiques, en particulier au niveau du poumon et du foie.

10. Antagoniste de la PTHrP selon l'une des revendications 7-9, **caractérisé en ce que** la composition est destinée au traitement de tumeurs malignes du cancer du rein, plus spécifiquement au traitement de tumeurs malignes solides.

11. Antagoniste de la PTHrP selon l'une des revendications 7-10, **caractérisé en ce que** l'anticorps anti-PTHrP est choisi parmi un anticorps humanisé, un anticorps humain, un anticorps chimérique, un anticorps obtenu à partir d'un hybridome ou un fragment de ces anticorps et/ou une forme modifiée dudit fragment.

12. Antagoniste de la PTHrP selon la revendication 11, **caractérisé en ce que** l'anticorps anti-PTHrP est un anticorps monoclonal.

## Claims

1. Use of at least one antagonist of PTHrP for the preparation of a pharmaceutical composition for treating preventatively or curatively a kidney cancer and in particular a renal cell carcinoma (RCC) in a mammal and in particular in a human being, said antagonist being an anti-PTHrP(34-53) antibody.

2. Use according to the preceding claim, wherein the composition is for treating papillary carcinoma (chromophiles), chromophobe cell carcinoma, Bellini carcinoma and unclassified renal cell carcinomas, or preferably clear cell carcinoma (CCC).

3. Use according to one of the preceding claims, wherein the composition is for inhibiting or decreasing the tumour growth and / or metastasis formation in kidney cancer and / or its metastatic developments, in particular in the lung and in the liver.

4. Use according to one of the preceding claims, wherein the composition is for treating malignant tumours of kidney cancer, in particular for treating solid malignant tumours.

5. Use according to one of claims 1 to 4, wherein the anti-PTHrP antibody is selected from a humanised antibody, a human antibody, a chimeric antibody, an antibody obtained from a hybridoma and a fragment thereof and/or a modified form of said fragment.

6. Use according to claim 5, wherein the anti-PTHrP antibody is a monoclonal antibody.

7. Antagonist of PTHrP for treating, preventatively or curatively, a kidney cancer, and in particular a renal cell carcinoma (RCC), in a mammal, and in particular in a human being, said antagonist being an anti-PTHrP(34-53) antibody.

8. Antagonist of PTHrP according to claim 7, wherein the composition is for treating papillary carcinoma (chromophiles), chromophobe cell carcinoma, Bellini carcinoma and unclassified renal cell carcinomas, or preferably clear cell carcinoma (CCC).

9. Antagonist of PTHrP according to one of claims 7-8, wherein the composition is for inhibiting or decreasing the tumour growth and / or metastasis formation in kidney cancer and / or its metastatic developments, in particular in the lung and in the liver.

10. Antagonist of PTHrP according to one of claims 7-9, wherein the composition is for treating malignant tumours of kidney cancer, in particular for treating solid malignant tumours.

11. Antagonist of PTHrP according to one of claims 7-10, wherein the anti-PTHrP antibody is selected from a humanised antibody, a human antibody, a chimeric antibody, an antibody obtained from a hybridoma and a fragment thereof and/or a modified form of said fragment.

12. Antagonist of PTHrP according to claim 11, wherein the anti-PTHrP antibody is a monoclonal antibody.

## Patentansprüche

1. Verwendung wenigstens eines Antagonisten des PTHrP bei der Herstellung einer pharmazeutischen Zusammensetzung zur präventiven oder kurativen Behandlung von Nierenkrebs und insbesondere des Nierenzellkarzinoms (NCC) beim Säuger und insbesondere beim Menschen, wobei besagter Antagonist ein Anti-PTHrP(34-53)-Antikörper ist.

2. Verwendung gemäß dem vorhergehenden Anspruch, **dadurch gekennzeichnet, dass** die Zusammensetzung zur Behandlung des papillären (chromophilen) Karzinoms, des chromophoben Zellkarzinoms, des Bellini-Karzinoms oder der nicht klassifizierten Nierenzellkarzinome oder vorzugsweise des klarzelligen Karzinoms (CCC) bestimmt ist.

3. Verwendung gemäß einem der vorhergehenden Ansprüche, **dadurch gekennzeichnet, dass** die Zusammensetzung zur Hemmung oder Verminderung des Tumorwachstums und/oder der Bildung von Metastasen bei Nierenkrebs und/oder seiner Metastasenentwicklungen insbesondere in der Lunge oder Leber bestimmt ist.

4. Verwendung gemäß einem der vorhergehenden Ansprüche, **dadurch gekennzeichnet, dass** die Zusammensetzung zur Behandlung maligner Tumoren des Nierenkrebses, spezifischer zur Behandlung solider maligner Tumoren, bestimmt ist.

5. Verwendung gemäß einem der Ansprüche 1 bis 4, **dadurch gekennzeichnet, dass** der Anti-PTHrP-Antikörper aus einem humanisierten Antikörper, einem humanen Antikörper, einem chimären Antikörper, einem aus einem Hybridom gewonnenen Antikörper oder einem Fragment dieser Antikörper und/oder einer modifizierten Form besagten Fragments ausgewählt ist.

6. Verwendung gemäß Anspruch 5, **dadurch gekennzeichnet, dass** der Anti-PTHrP-Antikörper ein monoklonaler Antikörper ist.

7. PTHrP-Antagonist zur präventiven oder kurativen Behandlung von Nierenkrebs und insbesondere des Nierenzellkarzinoms (NCC) beim Säuger und insbesondere beim Menschen, wobei besagter Antagonist ein Anti-PTHrP(34-53)-Antikörper ist.

8. PTHrP-Antagonist gemäß Anspruch 7, **dadurch gekennzeichnet, dass** die Zusammensetzung zur Behandlung des papillären (chromophilen) Karzinoms, des chromophoben Zellkarzinoms, des Bellini-Karzinoms oder der nicht klassifizierten Nierenzellkarzinome oder vorzugsweise des klarzelligen Karzinoms (CCC) bestimmt ist.

9. PTHrP-Antagonist gemäß einem der Ansprüche 7 bis 8, **dadurch gekennzeichnet, dass** die Zusammensetzung zur Hemmung oder Verminderung des Tumorwachstums und/oder der Bildung von Metastasen bei Nierenkrebs und/oder seiner Metastasenentwicklungen insbesondere in der Lunge oder Leber bestimmt ist.

10. PTHrP-Antagonist gemäß einem der Ansprüche 7 bis 9, **dadurch gekennzeichnet, dass** die Zusammensetzung zur Behandlung maligner Tumoren des Nierenkrebses, spezifischer zur Behandlung solider maligner Tumoren, bestimmt ist.

11. PTHrP-Antagonist gemäß einem der Ansprüche 7 bis 10, **dadurch gekennzeichnet, dass** der Anti-PTHrP-Antikörper aus einem humanisierten Antikörper, einem humanen Antikörper, einem chimären Antikörper, einem aus einem Hybridom gewonnenen Antikörper oder einem Fragment dieser Antikörper und/oder einer modifizierten Form besagten Fragments ausgewählt ist.

12. PTHrP-Antagonist gemäß Anspruch 11, **dadurch gekennzeichnet, dass** der Anti-PTHrP-Antikörper ein monoklonaler Antikörper ist.


### SEQUENCE LISTING

<110> université Louis Pasteur - INSERM - Hôpitaux Universitaires de Strasbourg
<120> Utilisation d'antagonistes de la pTHrP pour traiter le carcinome à cellules rénales
<130> B0148WO
<150> FR 02 08501
   <151> 2002-07-05
<160> 8
<170> PatentIn version 3.1
<210> 1
   <211> 21
   <212> DNA
   <213> artificial sequence
<220>
   <223> amorce
<400> 1
   atgcgacgga gactggttca g 21
<210> 2
   <211> 33
   <212> DNA
   <213> artificial sequence
<220>
   <223> amorce
<400> 2
   tcaatgcctc cgtgaatcga gctccagcga cgt 33
<210> 3
   <211> 22
   <212> DNA
   <213> artificial sequence
<220>
   <223> amorce
<400> 3
   aggaacagat cttcctgctg ca 22
<210> 4
   <211> 24
   <212> DNA
   <213> artificial sequence
<220>
   <223> amorce
<400> 4
   cacagctacg gtgagggacg ccag 24
<210> 5
   <211> 19
   <212> DNA
   <213> artificial sequence
<220>
   <223> amorce
<400> 5
   gcgtcgtgct gcccgtatg 19
<210> 6
   <211> 23
   <212> DNA
   <213> artificial sequence
<220>
   <223> amorce
<400> 6
   ttctgcacat ttgggtggtc ttc 23
<210> 7
   <211> 20
   <212> DNA
   <213> artificial sequence
<220>
   <223> amorce
<400> 7
   ggaaggtgaa ggtcggagtc 20
<210> 8
   <211> 19
   <212> DNA
   <213> artificial sequence
<220>
   <223> amorce
<400> 8
   gcagtgatgg catggactg 19